# EUROPEAN PATENT SPECIFICATION

(11) **EP 2 350 008 B1**
(45) Date of publication and mention of the grant of the patent: **05.09.2012**
(21) Application number: 09778756.8
(22) Date of filing: 29.09.2009
(51) Int. Cl.: C07D 213/64, C07D 213/65, C07D 213/68, A61K 31/44

(54) **METHOD FOR THE PREPARATION OF FUNCTIONALIZED TRIHALOMETHOXY SUBSTITUTED PYRIDINES**
VERFAHREN ZUR HERSTELLUNG FUNKTIONALISIERTER TRIHALOGENMETHOXYSUBSTITUIERTER PYRIDINE
PROCÉDÉ DE PRÉPARATION DE PYRIDINES SUBSTITUÉES PAR UN RÉSIDU TRIHALOGÉNOMÉTHOXY FONCTIONNALISÉES

(30) Priority: 09.10.2008 EP 08166247
(43) Date of publication of application: 03.08.2011
(73) Proprietor: Bayer CropScience AG, 40789 Monheim (DE); Centre National de la Recherche Scientifique, 75795 Paris (FR); Université de Strasbourg, 67000 Strasbourg (FR)
(72) Inventor: PAZENOK, Sergii, 42699 Solingen (DE); VORS, Jean-Pierre, 69110 Sainte Foy Les Lyon (FR); LEROUX, Fréderic, R., 67850 Herrlisheim (FR); MANTEAU, Baptiste, 17420 Saint Palais-sur-Mer (FR)
(74) Representative: Hildebrand, Steven
(86) International application number: PCT/EP2009/006986
(87) International publication number: WO 2010/040461

(56) References cited:
- WO-A-2006/055187
- US-A- 5 237 063
- E. J. BLANZ ET AL: "Carcinostatic Activity of Thiosemicarbazones of Formyl Heteroaromatic Compounds. VII. 2-Formylpyridine Derivatives Bearing Additional Ring Substituents" JOURNAL OF MEDICINAL CHEMISTRY, vol. 13, 1970, pages 1124-1130, XP002517476 cited in the application

## Description

The present invention pertains to a process for the preparation of functionalized trifluoromethoxypyridines comprising reacting hydroxypyridines with thiophosgene in the presence of a base; reacting the obtained chlorothionoformiates with elemental chlorine and finally converting trichloromethoxy pyridines to trifluoromethoxy pyridines.

Fluorine as a substituent in active ingredients plays a significant and increasingly important role. Currently about 15 % of the pesticides listed in the 13th edition of the Pesticide Manual contain at least one fluorine atom. The biggest group of fluorinated pesticides are the compounds containing a trifluoromethyl group (mainly in aromatic rings), followed by aromatic compounds containing an isolated fluorine atom (one and more). Only five pesticides contain OCF₃-groups. It was estimated that the number of fluorinated compounds currently under development represent some 35-50% of the all active ingredients under development as described in The pesticide manual, XIII edition; British crop protection council, 2003 or by Jeschke, P. ChemBioChem 2004, 5, 570-589.

Chlorination of aromatic methyl groups and exchange with fluorine can be used for the preparation of trifluoromethoxy substituted arenes as disclosed in Yagupol'skii, L. M. Dokl. Akad. Nauk SSSR 1955, 105, 100-102.

The displacement of the heavier by the lighter halogen can be brought about with anhydrous hydrogen fluoride or with antimony trifluoride in the presence of antimony pentachloride as described in Yagupol'skii, L. M.; Orda, V. V. Zh. Obshch. Khim. 1964, 34, 1979-1984 or in Yarovenko, N. N.; Vasil'eva, A. S. Zh. Obshch. Khim. 1958, 28, 2502-2504.

The side chain chlorination works well, when phosphorus pentachloride is employed at 200 °C as it becomes evident from Yagupol'skii, L. M.; Troitskaya, V. I. Zh. Obshch. Khim. 1961, 31, 915-924,or with elemental chlorine, photostimulated in refluxing tetrachloromethane, described in Louw, R.; Franken, P. W. Chem. Ind. 1977, 127-128.

When phenols are heated together with tetrachloromethane, anhydrous hydrogen fluoride and catalytic amounts of boron trifluoride up to 150 °C the corresponding (trifluoromethoxy)arenes are obtained as described in Feiring, A. E. J. Org. Chem. 1979, 44, 2907-2910 and in US 4,157,344*.*

Using elemental chlorine, the readily accessible, aryl chlorothionoformates can be cleanly converted into trichloromethyl aryl ethers, the precursors to (trifluoromethoxy)arenes as described in Yarovenko, N. N.; Vasil'eva, A. S. Zh. Obshch. Khim. 1958, 28, 2502-2504.

The latter compounds are also obtained when treated with molybdenum hexafluoride as it becomes evident from Mathey, F.; Bensoam, J. Tetrahedron Lett. 1973, 2253-2256.

When aryl formates are treated with sulfur tetrafluoride, (trifluoromethoxy)arenes are obtained in yields ranging from 9 - 81% as described in Sheppard, W. A. J. Org. Chem. 1964, 29, 1-11.

The oxidative fluorodesulfurization fluorination allows the conversion of dithiocarbonates (xanthogenates) with a huge excess of hydrogen fluoride-pyridine and 1,3-dibromo-5,5-dimethylhydantoin into (trifluoromethoxy)arenes in moderate to excellent yields as disclosed in Kuroboshi, M.; Suzuki, K.; Hiyama, T. Tetrahedron Lett. 1992, 33, 4173-4176; Kanie, K.; Tanaka, Y.; Suzuki, K.; Kuroboshi, M.; Hiyama, T. Bull. Chem. Soc. Japan 2000, 73, 471-484; Kuroboshi, M.; Kanie, K.; Hiyama, T. Adv. Synth. Catal. 2001, 343, 235-250 and Shimizu, M.; Hiyama, T. Angew. Chem. Int. Ed. 2005, 44, 214 - 231.

*O*-(Trifluoromethyl)dibenzofuranium salts convert alcohols and phenols into the corresponding trifluoromethoxy substituted derivatives as described in Umemoto, T.; Ishihara, S. J. Am. Chem. Soc. 1993, 115, 2156-2164; Umemoto, T.; Ishihara, S. Tetrahedron Lett. 1990, 31, 3579-3582; Umemoto, T.; Adachi, K.; Ishihara, S. J. Org. Chem. 2007, 72, 6905-6917 and Umemoto, T.; Adachi, K. J. Org. Chem. 1994, 59, 5692-5699.

However, actually no generally applicable procedure allows the preparation of pyridine trifluoromethyl ethers. Only some highly specific compounds are clearly described in the literature. In patents most often the generic structures are protected without any experimental details.

One 3-OCF₃-substituted pyridine has been prepared by oxidative desulfurization fluorination as described in Jiao, R.; Morriello, G.; Yang, L.; Goble, S. D.; Mills, S. G.; Pasternak, A.; Zhou, C.; Butora, G.; Kothandaraman, S.; Guiadeen, D.; Tang, C.; Moyes, C.; *WO2003092586* (Merck & Co., Inc., USA; Merck Sharp & Dohme Limited).

2,3-Dichloro-5-trifluoromethoxy pyridine was prepared according to Fuss, A.; Koch, V. Synthesis 1990, 604-608 by condensation of the corresponding 3-hydroxy pyridine with ClCF₂H followed by photochlorination and fluorination with SbF₃/SbCl₅.

OCF₃-Pyrimidines and -triazines were prepared by a radical chlorination and subsequent fluorination sequence as disclosed in EP-A-0445642. An OCF₃-chinoxalone was prepared by reaction of the phenole with difluorocarbene followed by fluorination with HF/pyridine as described in Morimoto, K.; Makino, K.; Sakata, G. J. Fluorine Chem. 1992, 59, 417-422.

Due to the importance of heterocyclic structures in agrochemical ingredients and the use of fluorine atoms and fluorinated groups in general, the possibility to prepare OCF₃-heterocycles will lead to new, so far unknown ingredients. For example the substitution of the OCF₃-phenyl ring in Indoxacarb by a OCF₃-pyridine ring may lead to more active compounds. Actually, no straightforward and generally applicable access to OCF₃-heterocycles is known.

Any unsubstituted 2-, 3- or 4-hydroxypyridine does not afford the desired OCF₃ pyridine by oxidative desulfurization fluorination, by alkylation/photochlorination or reaction with a difluorocarbene source (Br₂CF₂, BrClCF₂, Cl₂CF₂) followed by fluorination with HF/pyridine.

The synthesis of 3-[Fluoro(chloro)alkoxy]pyridines from halo- or amino-3-hydroxypiridines is disclosed by A. Fuss and V. Koch in Synthesis, 1990, 604-608. However, the synthesis described therein uses different reagents to transform the hydroxy group into a trihaloalkoxy group.

WO 2006/055187 discloses the synthesis of 2-bromo-5-trifluoromethoxypyridine (see example 99). However, the reaction steps according to the invention differ from the method described therein, since no thiophosgene is used in the prior art method.

E.J. Blanz et al discloses in J. Med. Chem. Vol. 13, 1970, 1124-1130 the synthesis of 2-methyl-5-trifluoromethoxypyridine. Neither the reagents used for the preparation nor the substituution pattern of the obtained pyridine equates to the present invention.

There is a strong need for a generally applicable process for the preparation of 2-, 3- or 4-OCF₃ pyridine building-blocks.

Furthermore, the general concept should start with an easily available compound and should contain few steps, allowing the synthesis of a variety of compounds, only depending on the reaction sequence. Therefore, all procedures relying on the use of different Freons (Br₂CF₂, BrClCF₂, Cl₂CF₂, ClCF₂H) had to be abandoned as these compounds are difficult to obtain as they are ozone depleting substances (ODS).

The problem has been solved according to the present invention by a process for the preparation of functionalized trihalomethoxypyridines of formula (I) 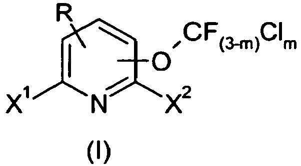 wherein
- X¹ and X²: are each individually selected from hydrogen, fluorine, chlorine or bromine, wherein at least one of X¹ and X² is different from hydrogen;
- R: is selected from hydrogen, halogen, C₁₋₁₂-alkyl-, C₃₋₈-cycloalkyl-, C₂₋₁₂-alkenyl-, C₂₋₁₂-alkynyl-, C₆₋₁₄-aryl-, C₇₋₁₉-arylalkyl- oder C₇₋₁₉-alkylaryl-groups, wherein each of the alkyl- cycloalkyl-, alkenyl-, alkynyl-, aryl-, aralkyl- and alkyryl-groups can be substituted by further residues selected from the group consisting of -R', -X, -OR', -SR', -NR'₂, -SiR'₃, -COOR', -CN and -CONR₂', wherein R' is hydrogen or a C₁₋₁₂-alkyl-residue
- m: is an integer 0
comprising the following steps (A) to (C):
(A) reacting hydroxypyridines according to formula (II) with thiophosgene in the presence of a base 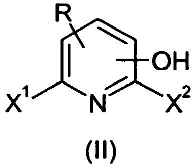
(B) reacting chlorothionoformiates of formula (III) with elemental chlorine; 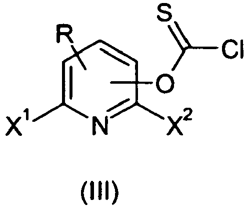
(C) treating the trichloromethoxy pyridines of the formula (IV) with a fluoride source to obtain trihalomethoxy pyridines of the formula (I)

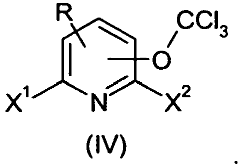

It was surprisingly found that the chlorothionoformiates of formula (III) can only be formed in presence of halogen atoms as protecting groups.

The dechlorination can be achieved, for instance, by treatment with hydrogen.

### General definitions

In connection with the present invention, the term halogens (X) comprises, unless otherwise defined, those elements which are chosen from the group consisting of fluorine, chlorine, bromine and iodine, fluorine, chlorine and bromine being preferably used and fluorine and chlorine being particularly preferably used.

Appropriately substituted groups can be mono- or polysubstituted, it being possible for the substituents in polysubstitutions to be identical or different.

Alkyl groups substituted with one or more halogen atoms (-X) are chosen, for example, from trifluoromethyl (CF₃), difluoromethyl (CHF₂), CF₃CH₂, ClCH₂, CF₃CCl₂ and CHF₂CCl₂.

Alkyl groups in connection with the present invention are, unless otherwise defined, linear, branched or cyclic hydrocarbon groups which can optionally exhibit one, two or more heteroatoms chosen from oxygen, nitrogen, phosphorus and sulphur. In addition, the alkyl groups according to the invention can optionally be substituted by additional groups chosen from -R', halogen (-X), alkoxy (-OR'), thioether or mercapto (-SR'), amino (-NR'₂), silyl (-SiR'₃), carboxyl (-COOR'), cyano (-CN), acyl (-(C=O)R') and amide (-CONR'₂) groups, R' being hydrogen or a C₁₋₁₂-alkyl group, preferably a C₂₋₁₀-alkyl group, particularly preferably a C₃₋₈-alkyl group, which can exhibit one or more heteroatoms chosen from nitrogen, oxygen, phosphorus and sulphur.

The definition C₁-C₁₂-alkyl comprises the biggest range defined herein for an alkyl group. Specifically, this definition comprises, for example, the meanings methyl, ethyl, n-propyl, isopropyl, n-butyl, isobutyl, sec-butyl and t-butyl, n-pentyl, n-hexyl, 1,3-dimethylbutyl, 3,3-dimethylbutyl, n-heptyl, n-nonyl, n-decyl, n-undecyl and n-dodecyl.

The definition cyclic C₃₋₁₂-alkyl groups comprises the biggest range defined herein for a cyclic alkyl group. Specifically, this definition comprises, for example, the meanings cyclopropyl, cyclobutyl, cyclopentyl, cyclohexyl, cycloheptyl and cyclooctyl.

Alkenyl groups in connection with the present invention are, unless otherwise defined, linear, branched or cyclic hydrocarbon groups which comprise at least one single unsaturation (double bond) and can optionally exhibit one, two or more single or double unsaturations or one, two or more heteroatoms chosen from oxygen, nitrogen, phosphorous and sulphur. In addition, the alkenyl groups according to the invention can optionally be substituted by additional groups chosen from -R', halogen (-X), alkoxy (-OR'), thioether or mercapto (-SR'), amino (-NR'₂), silyl (-SiR'₃), carboxyl (-COOR'), cyano (-CN), acyl (-(C=O)R') and amide (-CONR'₂) groups, R' being hydrogen or a C₁₋₁₂-alkyl group, preferably a C₂₋₁₀-alkyl group, particularly preferably a C₃₋₈-alkyl group, which can exhibit one or more heteroatoms chosen from nitrogen, oxygen, phosphorous and sulphur.

The definition C₂-C₁₂-alkenyl comprises the biggest range defined herein for an alkenyl group. Specifically, this definition comprises, for example, the meanings vinyl; allyl (2-propenyl), isopropenyl (1-methylethenyl); but-1-enyl (crotyl), but-2-enyl, but-3-enyl; hex-1-enyl, hex-2-enyl, hex-3-enyl, hex-4-enyl, hex-5-enyl; hept-1-enyl, hept-2-enyl, hept-3-enyl, hept-4-enyl, hept-5-enyl, hept-6-enyl; oct-1-enyl, oct-2-enyl, oct-3-enyl, oct-4-enyl, oct-5-enyl, oct-6-enyl, oct-7-enyl; non-1-enyl, non-2-enyl, non-3-enyl, non-4-enyl, non-5-enyl, non-6-enyl, non-7-enyl, non-8-enyl; dec-1-enyl, dec-2-enyl, dec-3-enyl, dec-4-enyl, dec-5-enyl, dec-6-enyl, dec-7-enyl, dec-8-enyl, dec-9-enyl; undec-1-enyl, undec-2-enyl, undec-3-enyl, undec-4-enyl, undec-5-enyl, undec-6-enyl, undec-7-enyl, undec-8-enyl, undec-9-enyl, undec-10-enyl; dodec-1-enyl, dodec-2-enyl, dodec-3-enyl, dodec-4-enyl, dodec-5-enyl, dodec-6-enyl, dodec-7-enyl, dodec-8-enyl, dodec-9-enyl, dodec-10-enyl, dodec-11-enyl; buta-1,3-dienyl, penta-1,3-dienyl.

Alkynyl groups in connection with the present invention are, unless otherwise defined, linear, branched or cyclic hydrocarbon groups which comprise at least one double unsaturation (triple bond) and can optionally exhibit one, two or more single or double unsaturations or one, two or more heteroatoms chosen from oxygen, nitrogen, phosphorous and sulphur. In addition, the alkynyl groups according to the invention can optionally be substituted by additional groups chosen from -R', halogen (-X), alkoxy (-OR'), thioether or mercapto (-SR'), amino (-NR'₂), silyl (-SiR'₃), carboxyl (-COOR'), cyano (-CN), acyl (-(C=O)R') and amide (-CONR'₂) groups, R' being hydrogen or a linear, branched or cyclic C₁₋₁₂-alkyl group which can exhibit one or more heteroatoms chosen from nitrogen, oxygen, phosphorous and sulphur.

The definition C₂-C₁₂-alkynyl comprises the biggest range defined herein for an alkynyl group. Specifically, this definition comprises, for example, the meanings ethynyl (acetylenyl); prop-1-ynyl and prop-2-ynyl.

Aryl groups in connection with the present invention are, unless otherwise defined, aromatic hydrocarbon groups which can exhibit one, two or more heteroatoms chosen from oxygen, nitrogen, phosphorous and sulphur and can optionally be substituted by additional groups chosen from -R', halogen (-X), alkoxy (-OR'), thioether or mercapto (-SR'), amino (-NR'₂), silyl (-SiR'₃), carboxyl (-COOR'), cyano (-CN), acyl (-(C=O)R') and amide (-CONR₂') groups, R' being hydrogen or a C₁₋₁₂-alkyl group, preferably a C₂₋₁₀-alkyl group, particularly preferably a C₃₋₈-alkyl group, which can exhibit one or more heteroatoms chosen from nitrogen, oxygen, phosphorous and sulphur.

The definition C₅₋₁₄-aryl comprises the biggest range defined herein for an aryl group having 5 to 14 atoms. Specifically, this definition comprises, for example, the meanings cyclopentadienyl, phenyl, cycloheptatrienyl, cyclooctatetraenyl, naphthyl and anthracenyl.

The definition C₅₋₁₄-aryl, preferably C₅₋₈-aryl groups exhibiting one, two or more heteroatoms chosen from oxygen, nitrogen, phosphorous and sulphur are chosen, for example, from the group consisting of 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-1-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

Arylalkyl groups (aralkyl groups) in connection with the present invention are, unless otherwise defined, alkyl groups substituted by aryl groups which can exhibit a C₁₋₈-alkylene chain and can be substituted in the aryl backbone or the alkylene chain by one or more heteroatoms chosen from oxygen, nitrogen, phosphorous and sulphur and optionally by additional groups chosen from -R', halogen (-X), alkoxy (-OR'), thioether or mercapto (-SR'), amino (-NR'₂), silyl (-SiR'₃), carboxyl (-COOR'), cyano (-CN), acyl (-(C=O)R') and amide (-CONR₂') groups, R' being hydrogen or a C₁₋₁₂-alkyl group, preferably a C₂₋₁₀-alkyl group, particularly preferably a C₃₋₈-alkyl group, which can exhibit one or more heteroatoms chosen from nitrogen, oxygen, phosphorous and sulphur.

The definition C₇₋₁₉-aralkyl group comprises the biggest range defined herein for an arylalkyl group with a total of 7 to 19 atoms in the backbone and alkylene chain. Preference is given to those C₇₋₁₉-aralkyl groups comprising 5 or 6 carbon atoms or heteroatoms in the aryl backbone and 1 to 8 carbon atoms in the alkylene chain. Specifically, this definition comprises, for example, the meanings benzyl and phenylethyl.

Alkylaryl groups (alkaryl groups) in connection with the present invention are, unless otherwise defined, aryl groups substituted by alkyl groups which can exhibit a C₁₋₈-alkylene chain and can be substituted in the aryl backbone or the alkylene chain by one or more heteroatoms chosen from oxygen, nitrogen, phosphorous and sulphur and optionally by additional groups chosen from -R', halogen (-X), alkoxy (-OR'), thioether or mercapto (-SR'), amino (-NR'₂), silyl (-SiR'₃), carboxyl (-COOR'), cyano (-CN), acyl (-(C=O)R') and amide (-CONR₂') groups, R' being hydrogen or a C₁₋₁₂-alkyl group, preferably a C₂₋₁₀-alkyl group, particularly preferably a C₃₋₈-alkyl group, which can exhibit one or more heteroatoms chosen from nitrogen, oxygen, phosphorous and sulphur.

The definition C₇₋₁₉-alkylaryl group comprises the biggest range defined herein for an alkylaryl group with a total of 7 to 19 atoms in the backbone and alkylene chain. Preference is given to those C₇₋₁₉-aralkyl groups comprising 5 or 6 carbon atoms or heteroatoms in the aryl backbone and 1 to 8 carbon atoms in the alkylene chain. Specifically, this definition comprises, for example, the meanings tolyl-, 2,3-, 2,4-, 2,5-, 2,6-, 3,4- or 3,5-dimethylphenyl.

The alkyl, alkenyl, alkynyl, aryl, alkylaryl and aralkyl groups can furthermore exhibit one or more heteroatoms which, unless otherwise defined, are chosen from nitrogen, oxygen, phosphorous and sulphur. The heteroatoms in this connection replace the carbon atoms indicated.

The compounds according to the invention can exist, if appropriate, as mixtures of different possible isomeric forms, in particular of stereoisomers, such as, e.g., E- and Z-isomers, threo-and erythro-isomers, and optical isomers, but, if appropriate, also tautomers. Both the E- and Z-isomers, as also the threo- and erythro-isomers, and also the optical isomers, any mixture of these isomers, and the possible tautomeric forms, are disclosed and claimed.

The functionalized trihalomethoxypyridines are compounds of formula (I) 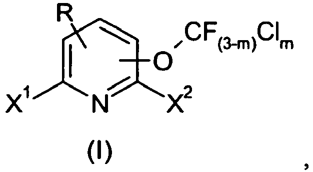 wherein
- R: is selected from hydrogen, halogen, C₁₋₁₂-alkyl-, C₃₋₈-cycloalkyl-, C₂₋₁₂-alkenyl-, C₂₋₁₂-alkynyl-, C₆₋₁₄-aryl-, C₇₋₁₉-arylalkyl- or C₇₋₁₉-alkylaryl-groups, wherein each of the alkyl- cycloalkyl-, alkenyl-, alkynyl-, aryl-, aralkyl- and alkyryl-groups can be substituted by further residues selected from the group consisting of -R', -X, -OR', -SR', -NR'₂, -SiR'₃, -COOR', -CN and -CONR₂', wherein R' is hydrogen or a C₁₋₁₂-alkyl-residue
- X¹ and X²: are each individually selected from hydrogen, fluorine, chlorine or bromine, wherein at least one of X¹ and X² is different from hydrogen;
- m: is an integer 0

In a preferred embodiment of the present invention
- R: is selected from hydrogen and C₁₋₄-alkyl;
- X¹ and X²: are each individually selected from hydrogen, chlorine or bromine, wherein at least one of X¹ and X² is different from hydrogen;
- m: is 0.

In a most preferred embodiment
- R: is hydrogen;

- X¹ and X²: are each individually selected from hydrogen and chlorine, wherein at least one of X¹ and X² is different from hydrogen;
- m: is 0.

**Step (A)** comprises reacting hydroxypyridines according to formula (II) with thiophosgene in the presence of a base according to the subsequent reaction scheme 1

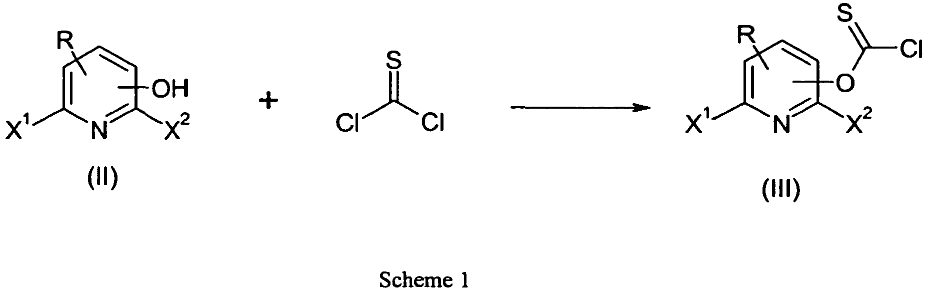

The preparation of chlorothionoformates according to step (A) was developed by Yarovenko and Vasil'eva on aromatic rings. Some examples describe the synthesis of thiocarbamates on pyridines, pyrimidines and thiophenes as disclosed in GB2122619, JP2000026432, JP2000026452, JP2000026464 and JP2000026419. However, the patent literature shows only functionalized pyridines. The transformation does not work with unfunctionalized hydroxy pyridines as shown in the comparative examples.

It has been found by the inventors that reactivity and yield are not affected as long as 2-, 6- or 2,6-dihalo-substituted pyridines are used. When the halogen-atoms are moved to the 3-, 4- or 5-position, respectively, the trichloromethoxylation (steps A and B) occurs with smaller yields asside with the formation of dimerization products. However, the subsequent trifluoromethoxylation (step C) fails completely. Only traces of chlorodifluoromethoxy-substituted compounds are obtained.

In a preferred embodiment of the present invention, a chlorohydroxypyridine according to formula (II) is treated with thiophosgene in presence of base affording the chlorothionoformate of formula (III).

Generally, step (A) is carried out in a solvent, such as chloroform, at a temperature ranging from -50 to 50°C, preferably -10 to 10 °C, most preferably from 0 °C to 5 °C with a base.

Bases which are suitable according to the present invention are for example primary, secondary and tertiary amines such as alkylamines, dialkylamines, trialkylamines, each of which may be alicyclic or open-chain; alkali metal and alkaline earth metal salts of aliphatic and/or aromatic carboxylic acids, such as acetates, propionates or benzoates; alkali metal and alkaline earth metal carbonates, hydrogencarbonates, phosphates, hydrogenphosphates and/or hydroxides; and metal alkoxides, in particular alkali metal or alkaline earth metal alkoxides, such as sodium methoxide, potassium methoxide, sodium ethoxide, magnesium methoxide, calcium ethoxide, sodium tert-butoxide, potassium tert-butoxide or alkali metal isoamylates. The base is preferably a carbonate, hydroxide or phosphate of lithium, sodium, potassium, calcium, magnesium or caesium. Particular preference is given to NaOH, KOH, potash and soda.

**Step (B)** comprises reacting chlorothionoformiates of formula (III) with elemental chlorine according to the subsequent reaction scheme 2

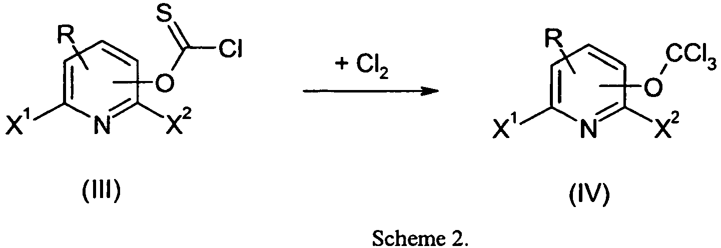

The conversion of chlorothionoformates according to formula (III) into a trichloromethoxy group according to step B (scheme 2) was described once by Yarovenko and Vasil'eva on aromatic rings, but never applied to heterocycles in particular not with pyridines. It is well known that the radical chlorination is strongly substrate dependant.

In a preferred embodiment of the invention the chlorothionoformiate (III) is immediately converted into the trichloromethoxy pyridines of the formula (IV) by addition of chlorine gas. The reactions is carried out in the same solvent after introduction of excess chlorine gas without isolation of the chlorothionoformiates (III).

**Step (C)** comprises converting trichloromethoxy pyridines of the formula (IV) into dichlorofluoromethoxy-, chlorodifluoromethoxy- or trifluoromethoxy pyridines of the formula (I) by chlorine/fluorine exchange according to the subsequent scheme 3

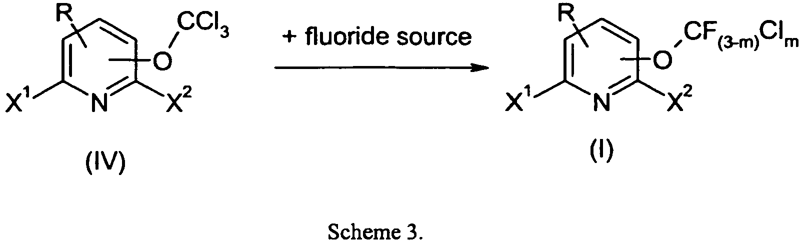

In reaction step C the trichloromethoxy pyridines of the formula (IV) are treated with a fluoride source optionally in the presence of a catalyst. The exchange of chlorine atoms by fluorine atoms was described by Hamprecht for the synthesis of pyrimidine and triazine derivatives. The required trichloromethoxy-substituted precursors were obtained by radical chlorination of the corresponding methoxy-derivatives as described in EP-A-0445642, EP-A-469462, DE-A-4024755 DE-A-4007683 DE-A-4128441 and DE-A-4105518.

However, this approach was never applied to pyridines.

Subsequently, the trichloromethoxy pyridines (IV) are submitted to a chlorine/fluorine exchange step (C) affording trifluoromethoxy pyridines, difluorochloromethoxy pyridines or dichlorofluoromethoxy pyridines. According to the invention between 1 and 4 equivalents, preferably between 1 and 2 equivalents of the fluoride source is used relative to trichloromethoxy equivalent. Optionally, step (C) can be carried out in presence of a catalyst which selectively affords the formation of the trifluoromethoxy pyridines. In a preferred embodiment SbF₃ is used as a fluoride source and SbCl₅ as catalyst without solvent. Preferably the ratio of SbF₃ and SbCl₅ is in the range of 2:1 to 20:1. More preferably the range is 5:1 to 15:1 and most preferably it is 13:1.

In yet another embodiment, the fluoride source in the reaction with the trichloromethoxy pyridines (IV) is selected from the group consisting of HF, HF*pyridine adduct, HF*amine adduct, KF, MoF₆, CoF₃, SbF₅.

Suitable catalysts are all compounds which increase the reaction rate of the chlorine/fluorine exchange. In a preferred embodiment, the catalyst is selected from SbCl₅ and BF₃.

In yet another embodiment, the chlorine/fluorine exchange step (C) is performed in a solvent, such as sulfolane.

The chlorine/fluorine exchange step (C) is performed at a temperature ranging from 25 °C to 150 °C. More preferably in the range from 120 °C to 150 °C.

A further aspect of the invention is the use of chlorine atoms in the alpha-position of the pyridine ring. Reactivity and yield of the process steps (A) and (B) are not affected when the Cl-atoms are replaced by Br- or F-atoms. However, the chlorine/fluorine exchange step (C) performs only satisfying in presence of Cl-atoms in the alpha-position of the pyridine ring. Therefore, in order to selectivly produce trifluoromethylpyridines it is important that the pyridines according to formula (IV) are chlorinated in the alpha-position to the pyridine nitrogen atom, i.e. in at least in 2- or 6- or in both positions.

Thus, in a most preferred embodiment of the invention the halogen exchange step (C) is carried out in presence of at least one chlorine atom protecting the trichloromethoxy pyridines of the formula (IV) (X=Cl).

Moreover, it has been found by the inventors that if the chlorine/fluorine exchange is performed without a catalyst, e.g. SbCl₅, or reduced amounts of it, for example 0.02 to 0.1 equivalent, albeit with a reduced amount of SbF₃, for example 0.5 to 1 equivalent, it can afford chlorodifluoromethoxy pyridines or dichlorofluoromethoxypyridines.

The chlorodifluoromethoxy- and trifluoromethoxypyridines can be further functionalized at any vacant position by means of metalation reactions using an organometallic compound or catalytic reactions using organometallic catalysts according to **Step (D)** affording derivatives (V).

An organometallic compound suitable for the present process must be able to undergo a halogen-metal exchange or a metalation of trifluoromethoxy pyridines of the formula I. In a preferred embodiment the organometallic compound is selected from the group consisting of Grignard reagents and lithiation reagents. Examples for suitable organometallic compounds are *n*-butyllithium, *tert*-butyllithium, phenyllithium, ethylmagnesium chloride, ethylmagnesium bromide, isopropylmagnesium chloride, isopropylmagnesium bromide and "magnesate" complexes. Magnesate complexes are for example lithium tri-*n*-butylmagnesate. In a preferred embodiment the metalation is carried out in a non-polar solvent. Preferably the solvent is selected from the group consisting of tetrahydrofuran, 1,4dioxane, cyclohexane, diethyl ether, *tert*-butyl methyl ether, diisopropyl, ether, benzene, toluene, and mixture thereof. After metalation, the organometallic intermediates are trapped with a suitable electrophile. In a preferred embodiment, suitable electrophiles are dry ice (carbon dioxide), bromine, 1,2-dibromotetrafluoroethane, CBr₄, 1,2-dibromoethane, iodine, hexachloroethane, 1,1,2-trichloro-1,2,2-trifluoroethane, N,N-dimethylformamide, N,N-dimethylacetamide, N,N-dimethylpropionamide, benzenesulfonylazide,*para*-tolylsulfonylazide etc. 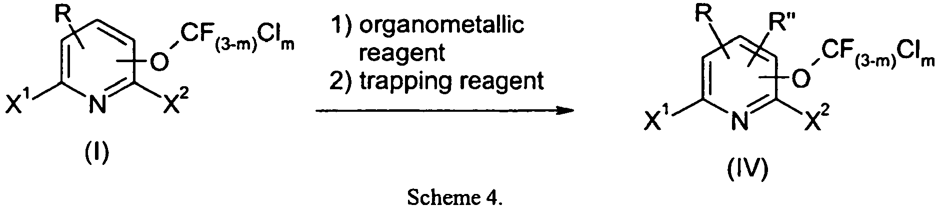 In formula (IV)
- R": is selected from halogen, -CHO, C₁₋₁₂-alkyl-, C₃₋₈-cycloalkyl-, C₂₋₁₂-alkenyl-, C₂₋₁₂-alkynyl-, C₆₋₁₄-aryl-, C₇₋₁₉-arylalkyl- or C₇₋₁₉-alkylaryl-groups, wherein each of the alkyl- cycloalkyl-, alkenyl-, alkynyl-, aryl-, aralkyl- and alkyryl-groups can be substituted by further residues selected from the group consisting of -R', -X, -OR', -SR', -NR'₂, -SiR'₃, -COOR', -CN and -CONR₂', wherein R' is hydrogen or a C₁₋₁₂-alkyl-residue, or OR', -SR', -NR'₂, -SiR'₃, -COOR', -CN and -CONR₂', wherein R' is hydrogen or a C₁₋₁₂-alkyl-residue.

In another embodiment the present invention relates to functionalized trihalomethoxypyridines of formula (I) 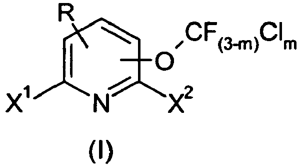 Wherein X¹, X², R an m are as defined above.

In a prefered embodiment embodiment the present invention relates to functionalized 3-trihalomethoxypyridines of formula (I-a).

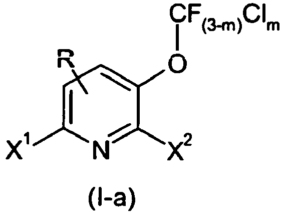

In a more preferred embodiment of the invention m in formula (I-a) is 0.

In a most preferred embodiment the functionalized 3-trihalomethoxypyridines of formula (I-a) is selected from the group consisting of 2-chloro-5-trifluoromethoxy isonicotinic acid, 3-trifluoromethoxy isonicotinic acid, 2-chloro-3-trifluoromethoxy isonicotinic acid, 4-amino-2-chloro-3-trifluoromethoxy pyridine, 4-amino-3-trifluoromethoxy pyridine, 2-chloro-4-iodo-3-trifluoromethoxy pyridine, 2-bromo-3-trifluoromethoxypyridine, 3-trifluoromethoxy picolinic acid, 2-chloro-5-trifluoromethoxy-4-trimethylsilyl pyridine, 6-chloro-3-trifluoromethoxy picolinic acid, 2-amino-3-trifluoromethoxypyridine, 2-chloro-4-iodo-5-trifluoromethoxypyridine, 2-chloro-6-iodo-5-trifluoromethoxypyridine, 2,6-dichloro-4-iodo-5-trifluoromethoxypyridine, 2-bromo-5-trifluoromethoxy pyridine, 5-trifluoromethoxy picolinic acid, 2-iodo-5-trifluoromethoxypyridine, 2-amino-5-trifluoromethoxy pyridine, 2-chloro-3-trifluoromethoxy-4-trimethylsilylpyridine, 6-chloro-5-trifluoromethoxy picolinic acid.

In a further embodiment the present invention relates to functionalized 2-trihalomethoxypyridines of formula (I-b).

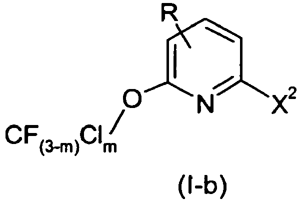

In a more preferred embodiment of the invention m in formula (I-b) is 0 and X² is Cl.

In a most preferred embodiment of the invention the functionalized 2-trihalomethoxypyridines of formula (I-b) selected from the group consisting of 6-chloro-2-trifluoromethoxy nicotinic acid, 2-Trifluoromethoxy nicotinic acid, 5-amino-2-chloro-6-trifluoromethoxypyridine, 3-amino-2-trifluoromethoxy pyridine, 2-chloro-5-iodo-6-trifluoromethoxy pyridine, 2-chloro-4-iodo-6-trifluoromethoxy pyridine, 2-chloro-6-trifluoromethoxy isonicotinic acid, 2-Trifluoromethoxy isonicotinic acid, 4-Amino-2-chloro-6-triftuoromethoxypyridine, 4-Amino-6-trifluoromethoxypyridine, 2-chloro-6-trifluoromethoxy-5-trimethylsilylpyridine, 2-chloro-3-iodo-6-trifluoromethoxypyridine, 2-chloro-6-trifluoromethoxynicotinic acid, 6-trifluoromethoxynicotinic acid, 3-amino-2-chloro-6-trifluoromethoxypyridine, 3-amino-6-trifluoromethoxypyridine, 3-*bis*(trimethylsilyl)amino-6-trifluoromethoxypyridine, 5-Amino-2-trifluoromethoxynicotinic acid, 2-Bromo-6-trifluoromethoxypyridine, 6-Trifluoromethoxypicolinic acid. 2-Amino-6-trifluoromethoxypyridine.

In a further embodiment the present invention relates to functionalized 4-trihalomethoxypyridines of formula (I-a).

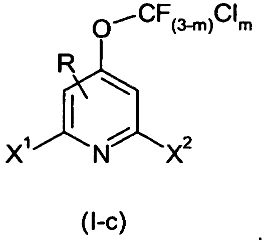

In a more preferred embodiment of the invention m in formula (I-c) is 0 and at least one of the residues X¹ and X² is chlorine.

In a most preferred embodiment the functionalized 3-trihalomethoxypyridines of formula (I-a) is 2-Chloro-4-trifluoromethoxynicotinic acid.

The present invention can be demonstrated by the following examples without limiting it thereto:

### Examples:

### -OCF₃ in 2-position:

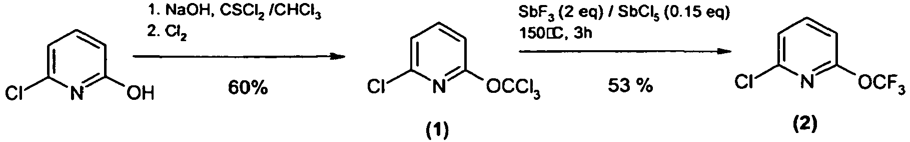

### 2-Chloro-6-trichloromethoxypyridine (1)

At 0 °C, thiophosgene (4.5 g, 3.0 mL, 39 mmol, 1 eq) in chloroform (24 mL) is added dropwise to a solution of 2-chloro-6-hydroxypyridine (5.0 g, 39 mmol) in aqueous sodium hydroxide (5%, 34 mL). The reaction mixture is vigorously stirred for 2 h at 0 °C before being extracted with chloroform (3 × 20 mL). The combined organic layers are washed with diluted hydrochloric acid (1 N, 20 mL) and water (20 mL), dried over sodium sulfate before being filtrated. At 25 °C, the filtrate is then saturated with chlorine until the reaction mixture begins to warm up. After 2 h at 25 °C, another excess of chlorine is added until a yellow solution is obtained. After 24 h at 25 °C excess of chlorine is removed by a stream of Ar gas and the solvent is evaporated. The crude pale yellow oil is distilled under vacuum to afford pure 2-chloro-6-trichloromethoxypyridine (1, 5.9 g, 23 mmol, 60%) as colorless crystals; b.p. 80-82 °C / 1 mbar; m.p. 37-39 °C.
¹H NMR (CDCl₃, 300 MHz): δ = 7.70 (t, *J* = 7.9 Hz, 1 H), 7.19 (d, *J* = 7.7 Hz, 1 H), 7.02 (d, *J* = 8.1 Hz, 1 H). - ¹³C NMR (CDCl₃, 75 MHz): δ = 151.7, 148.8, 141.5, 122.2, 112.8, 112.5. - MS(EI): m/z = 246 [M⁺], 211 [M⁺-Cl].

### 2-Chloro-6-trifluoromethoxypyridine (2)

2-Chloro-6-trichloromethoxypyridine (**1**, 5.9 g, 23.8 mmol) was added dropwise to a molten mixture of SbF₃ (8.7 g, 47.7 mmol, 2.0 eq) and SbCl₅ (1.0 g, 0.45 mL, 3.6 mmol, 0.15 eq) at 120 °C and stirred for 3 h at 150 °C. GC monitoring indicated 100% conversion and disappearance of byproduct OCF₂Cl. The mixture was then cooled to 0 °C and dissolved in dichloromethane (100 mL). The solution was quenched with saturated aqueous solution of sodium hydrogencarbonate (100 mL) and potassium fluoride (20%, 50 mL), the aqueous layer was extracted with dichloromethane (2 × 50 mL). The combined organic layers were dried over sodium sulfate and the solvent was distilled. The crude product was distilled under vacuum to afford pure 2-chloro-6-trifluoromethoxypyridine (**2**, 2.5 g, 10.1 mmol, 53%) as a colorless oil; b.p. 42-44 °C / 20 mbar. - ¹H NMR (CDCl₃, 300 MHz): δ = 7.67 (t, *J* = 7.9 Hz, 1 H), 7.18 (d, *J* = 7.7 Hz, 1 H), 6.87 (d, *J* = 8.0 Hz, 1 H). - ¹³C NMR (CDCl₃, 75 MHz): δ = 155.6, 149.3, 141.9, 122.2, 119.8 (q, *J* = 262 Hz), 111.8. - MS(EI): m/z = 197 [M⁺], 162 [M⁺-Cl].

### -OCF₃ in 3-position:

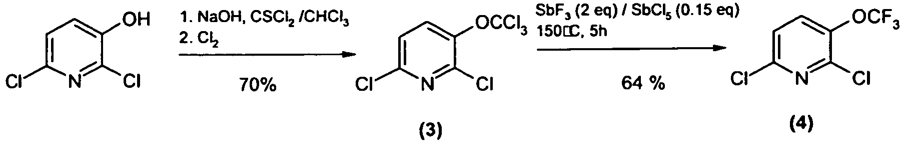

### 2,6-Dichloro-3-trichloromethoxypyridine (3)

At 0 °C, thiophosgene (4.3 g, 2.9 mL, 36.6 mmol, 1 eq) in chloroform (22 mL) is added dropwise to a solution of 2,6-dichloro-3-hydroxypyridine (6.0 g, 36.6 mmol) in aqueous sodium hydroxide (5%, 32 mL). The reaction mixture is vigorously stirred for 2 h at 0 °C before being extracted with chloroform (3 × 20 mL). The combined organic layers are washed with diluted hydrochloric acid (1 N, 20 mL) and water (20 mL), dried over sodium sulfate before being filtrated. At 25 °C, the filtrate is then saturated with chlorine until the reaction mixture begins to warm up. After 2 h at 25 °C, another excess of chlorine is added until a yellow solution is obtained. After 24 h at 25 °C excess of chlorine is removed by a stream of Ar gas and the solvent is evaporated. The crude pale yellow oil is distilled under vacuum to afford pure 2,6-dichloro-3-trichloromethoxypyridine (3, 7.1 g, 25.4 mmol, 70%) as colorless crystals; b.p. 78-81 °C / 0.5 mbar; m.p. 41-43 °C.
¹H NMR (CDCl₃, 300 MHz): δ = 8.02 (d, *J* = 8.5 Hz, 1 H), 7.37 (d, *J* = 8.5 Hz, 1 H). - ¹³C NMR (CDCl₃, 75 MHz): δ = 144.7, 143.4, 132.9, 123.6, 115.0, 112.2. - MS(EI): m/z = 281 [M⁺], 245 [M⁺-Cl].

### 2,6-Dichloro-3-trifluoromethoxypyridine (4)

2,6-Dichloro-3-trichloromethoxypyridine (**3**, 4.0 g, 14.3 mmol) is added dropwise to a molten mixture of SbF₃ (5.2 g, 28.7 mmol, 2.0 eq) and SbCl₅ (0.6 g, 0.3 mL, 2.1 mmol, 0.15 eq) at 120 °C and stirred for 5 h at 150 °C. GC monitoring indicated 100% conversion and disappearance of byproduct OCF₂Cl. The mixture is then cooled to 0 °C and dissolved in dichloromethane (100 mL). The solution is quenched with saturated aqueous solution of sodium hydrogencarbonate (100 mL) and potassium fluoride (20%, 50 mL), the aqueous layer is extracted with dichloromethane (2 × 50 mL). The combined organic layers are dried over sodium sulfate and the solvent is distilled. The crude product is distilled under vacuum to afford pure 2,6-dichloro-3-trifluoromethoxypyridine (**4**, 2.2 g, 9.3 mmol, 64%) as a colorless oil; b.p. 65-66 °C / 20 mbar.
¹H NMR (CDCl₃, 300 MHz): δ = 7.62 (d, *J* = 8.5 Hz, 1 H), 7.26 (d, *J* = 8.5 Hz, 1 H). - ¹³C NMR (CDCl₃, 75 MHz): δ = 148.2, 144.5, 141.8, 133.3, 124.4, 120.7 (q, *J* = 261 Hz). - MS(EI): m/z = 231 [M⁺], 195 [M⁺-Cl].

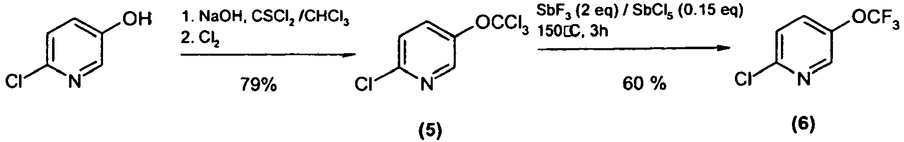

### 2-Chloro-5-trichloromethoxypyridine (5)

At 0 °C, thiophosgene (4.3 g, 2.9 mL, 37 mmol, 1 eq) in chloroform (22 mL) is added dropwise to a solution of 2-chloro-5-hydroxypyridine (4.8 g, 37 mmol) in aqueous sodium hydroxide (5%, 32 ml). The reaction mixture is vigorously stirred for 2 h at 0 °C before being extracted with chloroform (3 × 20 mL). The combined organic layers are washed with diluted hydrochloric acid (1 N, 20 mL) and water (20 mL), dried over sodium sulfate before being filtrated. At 25 °C, the filtrate was then saturated with chlorine until the reaction mixture begins to warm up. After 2 h at 25 °C, another excess of chlorine is added until a yellow solution is obtained. After 24 h at 25 °C excess of chlorine is removed by a stream of Ar gas and the solution is evaporated. The crude pale yellow oil is distilled under vacuum to afford pure 2-chloro-5-trichloromethoxypyridine (**5**, 7.1 g, 28.9 mmol, 79%) as colorless crystals; b.p. 71-73 °C / 0.5 mbar; m.p. 38-41 °C.
¹H NMR (CDCl₃, 300 MHz): δ = 8.47 (d, *J* = 2.8 Hz, 1 H), 7.75 (dd, *J* = 8.7, 2.8 Hz, 1 H), 7.42 (d, *J* = 8.7 Hz, 1 H). - ¹³C NMR (CDCl₃, 75 MHz): δ = 149.4, 147.9, 144.2, 133.0, 125.2, 112.2. - MS(EI): m/z = 246 [M⁺], 211 [M⁺-Cl].

### 2-Chloro-5-trifluoromethoxypyridine (6)

2-Chloro-5-trichloromethoxypyridine (**5**, 7.4 g, 30.0 mmol) is added dropwise to a molten mixture of SbF₃ (10.7 g, 60.0 mmol, 2.0 eq) and SbCl₅ (1.4 g, 0.6 mL, 4.6 mmol, 0.15 eq) at 120 °C and stirred for 3 h at 150 °C. GC monitoring indicates 100% conversion and disappearance of byproduct OCF₂Cl. The mixture is then cooled to 0 °C and dissolved in dichloromethane (100 mL). The solution is quenched with saturated aqueous solution of sodium hydrogencarbonate (100 mL) and potassium fluoride (20%, 50 mL), the aqueous layer is extracted with dichloromethane (2 × 50 mL). The combined organic layers are dried over sodium sulfate and the solvent is distilled. The crude product is distilled under vacuum to afford pure 2-chloro-5-trifluoromethoxypyridine (**6**, 3.6 g, 18.0 mmol, 60%) as a colorless oil; b.p. 41-43 °C / 20 mbar.
¹H NMR (CDCl₃, 300 MHz): δ = 8.35 (s, 1 H), 7.55 (d, *J* = 8.7 Hz, 1 H), 7.41 (d, *J* = 8.7 Hz, 1 H). - ¹³C NMR (CDCl₃, 75 MHz): δ = 149.3, 142.7, 140.1, 131.3, 125.1, 120.2 (q, *J=* 259 Hz). - MS(EI): m/z = 197 [M⁺], 162 [M⁺-Cl].

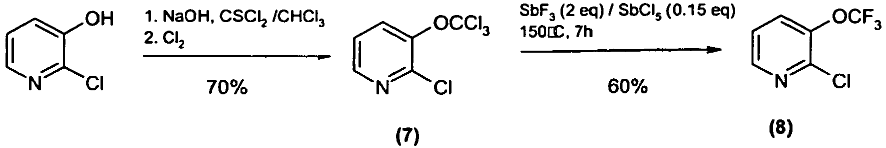

### 2-Chloro-3-trichloromethoxypyridine (7)

At 0 °C, thiophosgene (22.2 g, 14.8 mL, 0.19 mol, 1 eq) in chloroform (115 mL) is added dropwise to a solution of 2-chloro-3-hydroxypyridine (25.0 g, 0.19 mol) in aqueous sodium hydroxide (5%, 160 mL). The reaction mixture is vigorously stirred for 2 h at 0 °C before being extracted with chloroform (3 × 100 mL). The combined organic layers are washed with diluted hydrochloric acid (1 N, 100 mL) and water (100 mL), dried over sodium sulfate before being filtrated. At 25 °C, the filtrate is then saturated with chlorine until the reaction mixture begins to warm up. After 2 h at 25 °C, another excess of chlorine is added until a yellow solution is obtained. After 24 h at 25 °C excess of chlorine removed by a stream of Ar gas and the solvent is evaporated. The crude pale yellow oil is distilled under vacuum to afford pure 2-chloro-3-trichloromethoxypyridine (7, 32.9 g, 0.13 mol, 70%) as a colorless oil; b.p. 78-80 °C / 4 mbar.
¹H NMR (CDCl₃, 300 MHz): δ = 8.36 (dd, *J=* 4.7, 1.6 Hz, 1 H), 8.05 (dd, *J* = 8.2, 1.6 Hz, 1 H), 7.35 (dd, *J*= 8.2, 4.7 Hz, 1 H). - ¹³C NMR (CDCl₃, 75 MHz): δ = 146.7, 146.5, 145.5, 131.0, 123.5,112.0. -MS(EI): m/z = 246 [M⁺], 210 [M⁺-Cl].

### 2-Chloro-3-trifluoromethoxypyridine (8)

2-Chloro-3-trichloromethoxypyridine (**7**, 32.9 g, 0.13 mol) is added dropwise to a molten mixture of SbF₃ (46.5 g, 0.26 mol, 2.0 eq) and SbCl₅ (5.8 g, 2.5 mL, 19.5 mmol, 0.15 eq) at 120 °C and stirred for 7 h at 150 °C. GC monitoring indicates 100% conversion and disappearance of byproduct OCF₂Cl. The mixture is then cooled to 0 °C and dissolved in dichloromethane (300 mL). The solution is quenched with a saturated aqueous solution of sodium hydrogencarbonate (300 mL) and potassium fluoride (20%, 150 mL), the aqueous layer is extracted with dichloromethane (2 × 150 mL). The combined organic layers are dried over sodium sulfate and the solvent is distilled. The crude product is distilled under vacuum to afford pure 2-chloro-3-trifluoromethoxypyridine (**8**, 14.9 g, 75.6 mmol, 60%) as a colorless oil; b.p. 57-59 °C / 19 mbar.
¹H NMR (CDCl₃, 300 MHz): δ = 8.37 (dd, *J=* 4.7, 1.5 Hz, 1 H), 7.68 (dt, *J=* 8.1, 1.5 Hz, 1 H), 7.41 (dd, *J* = 8.1, 4.7 Hz, 1 H). - ¹³C NMR (CDCl₃, 75 MHz): δ = 147.4, 144.9, 142.2, 130.8, 123.2, 120.2 (q, *J*= 260 Hz). - MS(EI): m/z = 197 [M⁺], 162 [M⁺-Cl].

### OCF₃ in 4-position:

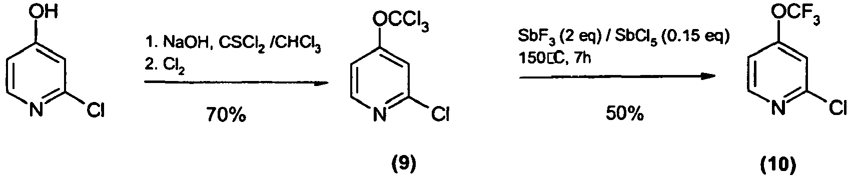

### 2-Chloro-4-trichloromethoxypyridine (9)

At 0 °C, thiophosgene (2.5 g, 1.7 mL, 21.5 mmol, 1 eq) in chloroform (15 mL) is added dropwise to a solution of 2-chloro-4-hydroxypyridine (2.8 g, 21.5 mmol) in aqueous sodium hydroxide (5%, 20 mL). The reaction mixture is vigorously stirred for 2 h at 0 °C before being extracted with chloroform (3 × 10 mL). The combined organic layers are washed with dilute hydrochloric acid (1 N, 20 mL) and water (20 mL), dried over sodium sulfate before being filtrated. At 25 °C, the filtrate is then saturated with chlorine until the reaction mixture begins to warm up. After 2 h at 25 °C, another excess of chlorine was added until a yellow solution is obtained. After 24 h at 25 °C excess of chlorine removed by a stream of Ar gas and the solution is evaporated. The crude pale yellow oil is distilled under vacuum to afford pure 2-chloro-4-trichloromethoxypyridine (**9**, 3.7 g, 15.0 mmol, 70%) as a colorless oil; b.p. 79-81 °C / 1 mbar.
¹H NMR (CDCl₃, 300 MHz): δ = 8.36 (d, *J* = 5.7 Hz, 1 H), 7.28 (d, *J* = 2.1 Hz, 1 H), 7.21 (dd, *J* = 5.7, 2.1 Hz, 1 H). - ¹³C NMR (CDCl₃, 75 MHz): δ = 159.3, 152.4, 150.8, 116.7, 115.9, 110.0. - MS(EI): m/z = 246 [M⁺], 211 [M⁺-Cl].

### 2-Chloro-4-trifluoromethoxypyridine (10)

2-Chloro-4-trichloromethoxypyridine (**9**, 3.5 g, 14.2 mmol) was added dropwise to a molten mixture of SbF₃ (5.0 g, 28.4 mmol, 2.0 eq) and SbCl₅ (635 mg, 0.27 mL, 2.1 mmol, 0.15 eq) at 120 °C and stirred for 7 h at 150 °C. GC monitoring indicated 100% conversion and disappearance of byproduct OCF₂Cl. The mixture was then cooled to 0 °C and dissolved in dichloromethane (100 mL). The solution was quenched with saturated aqueous solution of sodium hydrogencarbonate (100 mL) and potassium fluoride (20%, 75 mL), the aqueous layer was extracted with dichloromethane (2 × 50 mL). The combined organic layers were dried over sodium sulfate and the solvent was distilled. The crude product was distilled under vacuum to afford pure 2-chloro-4-trifluoromethoxypyridine (**10**, 1.4 g, 7.1 mmol, 50%) as a colorless oil; b.p. 39-41 °C / 20 mbar.
¹H NMR (CDCl₃, 300 MHz): δ = 8.30 (d, *J*= 5.4 Hz, 1 H), 7.36 (d, *J*= 1.6 Hz, 1 H), 7.24 (dd, *J* = 5.4, 1.6 Hz, 1 H). - ¹³C NMR (CDCl₃, 75 MHz): δ = 157.1, 152.2, 150.1, 114.8, 113.1, 120.3 (q, *J* = 260 Hz). - MS(EI): m/z = 197 [M⁺], 162 [M⁺-Cl].

### Example of Dechlorination

### 3-Trifluoromethoxyisonicotinic acid

At 25 °C, palladium (10% on charcoal, 200 mg) is added with stirring to a solution of 2-chloro-3-trifluoromethoxyisonicotinic acid (660 mg, 2.7 mmol) and ammonium formate (290 mg, 4.6 mmol, 1.7 eq) in methanol (5 mL). The reaction mixture is stirred for 16 h at 55 °C before being filtrated under suction and the filtrate evaporated. The residue is partitioned between ethyl acetate (2× 10 mL) and 2.0 M hydrochloric acid (15 mL). The combined organic layers are dried over sodium sulfate before being evaporated to afford pure 3-trifluoromethoxyisonicotinic acid (450 mg, 3.0 mmol, 81%) as a white powder; m.p. 181-183 °C.
¹H NMR (CD₃OD, 300 MHz): δ = 8.42 (d, *J* = 4.9 Hz, 1 H), 8.41 (s, 1 H), 7.46 (d, *J* = 4.9 Hz, 1 H). - ¹³C NMR (CD₃OD, 75 MHz): δ = 164.0, 148.7, 144.5, 143.0, 134.0, 124.7, 120.4 (q, *J* = 258 Hz). - MS(EI): m/z = 206 [M⁺].

### Comparative example 1:

Attempted oxidative desulfurization fluorination of pyridine xanthogenates

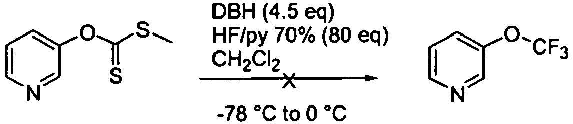

The preparation of OCF₃ pyridines using unsubstituted hydroxypyridines applying the oxidative desulfurization fluorination fails.

### S-methyl-O-pyridin-3-ylcarbonodithioate (11)

At 0 °C, 3-hydroxypyridine (980 mg, 10 mmol, 1 eq) in *N,N-*dimethylacetamide (10 mL) is added to a suspension of sodium hydride (60%, 1.2 g, 30 mmol, 3 eq) in *N,N-*dimethylacetamide (10 mL). The reaction mixture is then vigorously stirred 1 h at 25 °C and 30 min at 55°C. At 0 °C, carbon disulfide (3.8 g, 3 mL, 50 mmol, 5 eq) is added dropwise and the reaction mixture is allowed to reach 25 °C during 90 min. At 0 °C, iodomethane (3.0 g, 1.3 mL, 20 mmol, 2 eq is then added dropwise and the reaction mixture is allowed to reach 25 °C during 30 min. The reaction mixture is filtered through silica gel with dichloromethane and the solvent is evaporated. The crude product is purified by chromatography on silica gel using ethyl acetate/cyclohexane (1:4) as eluent, which affords pure *S*-methyl-*O*-pyridin-3-ylcarbonodithioate (**11**, 1.35 g, 7,3 mmol, 73%) as a dark red oil.
¹H NMR (CDCl₃, 300 MHz): δ = 8.45 (dd, *J*= 4.7, 1.4 Hz, 1 H), 8.34 (d, *J* = 2.6 Hz, 1 H), 7.36 (ddd, *J* = 8.3, 1.4, 2.6 Hz, 1 H), 7.29 (dd, *J* = 8.3, 4.7 Hz, 1 H), 2.58 (s, 3 H). - ¹³C NMR (CDCl₃, 75 MHz): δ = 215.4, 151.2, 147.6, 144.1, 129.9, 123.9, 20.1. - MS(EI): m/z = 186 [M⁺].

### S-methyl-O-pyridin-4-ylcarbonodithioate (12)

At 0 °C, 4-hydroxypyridine (4.8 g, 50 mmol, 1 eq) in *N,N*-dimethylformamide (40ml) is added to a suspension of sodium hydride (60%, 8.0 g, 0.15 mol, 3 eq) in *N,N*-dimethylacetamide (40 mL). The reaction mixture is then stirred vigorously 1 h at 25 °C and 30 min at 55°C. At 0 °C, carbon disulfide (18.9 g, 15 mL, 0.25 mol, 5 eq) is added dropwise and the reaction mixture is allowed to reach 25 °C during 16 h. At 0 °C, iodomethane (13.7g, 6.0 mL, 0.1 mol, 2 eq) is then added dropwise and the reaction mixture is allowed to reach 25 °C during 60 min. After addition of ice water (100 mL), the product precipitates. The mixture is cooled over night at 0°C before being filtrated. The crude product is purified by chromatography on silica gel using ethyl acetate/cyclohexane (1:4) as eluent which affords pure *S*-methyl-*O*-pyridin-4-ylcarbonodithioate (**12**, 7.4 g, 39,9 mmol, 80%) as a yellow powder; m.p. 132-134 °C.
¹H NMR (CDCl₃, 300 MHz): δ = 8.53 (d, *J* = 8.2 Hz, 2 H), 6.24 (d, *J* = 8.2 Hz, 2 H), 2.72 (s, 3 H). - ¹³C NMR (CDCl₃, 75 MHz): δ = 202.3, 180.7, 135.4 (2 C), 118.1 (2 C), 21.6. - MS(EI): m/z = 186 [M⁺].

### S-methyl-O-pyridin-2-ylcarbonodithioate (13)

At 0 °C, 2-hydroxypyridine (4.8 g, 50 mmol, 1 eq) in *N,N*-dimethylformamide (40 mL) is added to a suspension of sodium hydride (60%, 8.0 g, 0.15 mol, 3 eq) in *N,N*-dimethylacetamide (40mL). The reaction mixture is then stirred vigorously 1 h at 25 °C and 30 min at 55°C. At 0 °C, carbon disulfide (18.9 g, 15 mL, 0.25 mol, 5 eq) is added dropwise and the reaction mixture is allowed to reach 25 °C during 16 h. At 0 °C, iodomethane (13.7 g, 6.0 mL, 0.1 mol, 2 eq) is then added dropwise and the reaction mixture is allowed to reach 25 °C during 60 min. After addition of water (100 mL) the organic phase is separated and the aqueous layer is extracted with ethyl acetate (3 × 50 mL). The combined organic layers are dried over sodium sulfate before evaporating the solvent. The crude product is purified by chromatography on silica gel using ethyl acetate/cyclohexane (1 : 4) as eluent which affords pure *S*-methyl-*O*-pyridin-2-ylcarbonodithioate (**13**, 1.9 g, 10 mmol, 20%) as a yellow powder; m.p. 54-56 °C.
¹H NMR (CDCl₃, 300 MHz): δ = 8.08 (ddd, *J =* 7.3, 1.9, 0.6 Hz, 1 H), 7.24 (ddd, *J =* 9.3, 6.5, 1.9 Hz, 1 H), 6.51 (ddd, *J* = 9.3, 1.0, 0.6 Hz, 1 H), 6.16 (ddd, *J* = 7.5, 6.5, 1.0 Hz, 1 H), 2.67 (s, 3 H). - ¹³C NMR (CDCl₃, 75 MHz): δ = 209.1, 161.0, 140.0, 134.9, 122.7, 106.6, 23.3. - C₇H₇NS₂O (185.27): calcd. (%) C 45.33, H 3.78, N 7.55; found C 45.36, H 3.99, N 7.47.

### 3-trifluoromethoxypyridine (failed reaction)

At -78 °C, hydrogen fluoride in pyridine (70%, 80 ml, 0.8 mol, 80 eq) is added dropwise to a suspension of 1,3-dibromo-5,5-dimethylhydantoin (12.8 g, 45 mmol, 4.5 eq) in dichloromethane (60 mL). The mixture is then vigorously stirred for 30 min at -78 °C before *S*-methyl-*O*-pyridin-3-ylcarbonodithioate (**11**, 1.85 g, 10 mmol) in dichloromethane (40 mL) is added dropwise. The reaction mixture is then vigorously stirred at -5 °C during 2 h before being diluted with diethylether (40 mL) at this temperature and quenched with a cold solution of sodium bicarbonate and sodium bisulfite until the red color disappears. At 0 °C, the pH is adjusted to 10-11 with a sodium hydroxide solution (5.0 N) and the layers are separated. The organic layer is dried over sodium sulfate before being evaporated to afford a crude yellow oil which does not contain any product according to NMR and mass analysis.

### Comparative example 2:

### Alkylation of hydroxypyridines with freons:

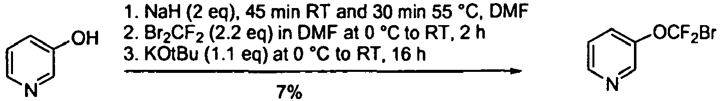

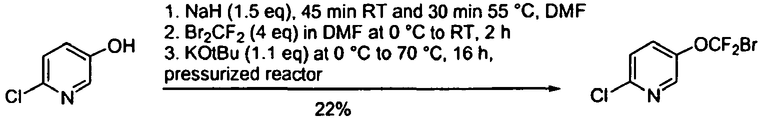

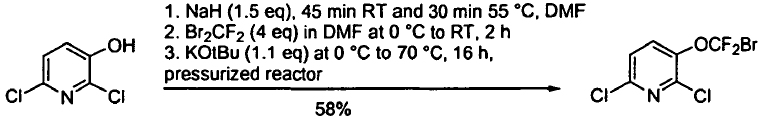

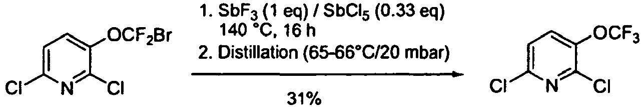

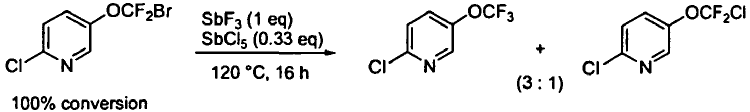

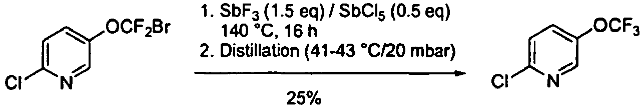

The alkylation of hydroxypyridines with fluorocarbenes works better with chlorinated hydroxypyridines. Subsequent fluorination with SbF₃/SbCl₅ affords the desired OCF₃-pyridines. However, these freons are no longer available as they are ozone depleting substances (ODS).

### 3-(Bromodifluoromethoxy)-pyridine (14)

At 0 °C, 3-hydroxypyridine (5 g, 52.6 mmol) in DMF (20 mL) is added to a suspension of sodium hydride (60%, 4.2 g, 105 mmol, 2 eq) in DMF (20 mL). The reaction mixture is then vigorously stirred for 1 h at 25 °C and 30 min at 55°C. At 0 °C, a solution of dibromodifluoromethane (25.2 g, 11 mL, 115.6 mmol, 2.2 eq) in DMF (20 mL) is added dropwise and the reaction mixture is allowed to reach 25 °C during 2 h. At 0 °C, potassium *tert-*butoxide (6.3 g, 57.8 mmol, 1.1 eq) is then added portion wise and the reaction mixture is allowed to reach 25 °C during 16 h. After addition of water (100 mL) the organic phase is separated and the aqueous layer is extracted with ethyl acetate (3 × 50 mL). The combined organic layers are dried over sodium sulfate before being evaporated. The crude product is purified by chromatography on silica gel using ethyl acetate / cyclohexane (5 : 95) as eluent which affords pure 3-(bromodifluoromethoxy)pyridine (**14**, 840 mg, 3.7 mmol, 7%) as a red oil.
¹H NMR (CDCl₃, 300 MHz): δ = 8.51 (dd, *J =* 4.7, 1.3 Hz, 2 H), 7.51 (ddd, *J =* 8.3, 4.7, 1.3 Hz, 1 H), 7.30 (dd, *J* = 8.3, 4.7 Hz, 1 H). - ¹³C NMR (CDCl₃, 75 MHz): δ = 148.5, 147.8, 143.4, 128.8, 124.1, 114.4 (t, *J* = 115 Hz). - MS(EI): m/z = 224 [M⁺], 144 [M⁺-Br].

### 3-(Bromodifluoromethoxy)-2,6-dichloropyridine (15)

At 0 °C, sodium hydride (60%, 1.8 g, 45.7 mmol, 1.5 eq) is added portion wise to a solution of 2,6-dichloro-3-hydroxypyridine (5.0 g, 30.5 mmol) in DMF (50 mL). The reaction mixture is then vigorously stirred for 1 h at 25 °C and 30 min at 55°C. At 0 °C, a solution of dibromodifluoromethane (25.7 g, 11.2 mL, 122 mmol, 4 eq) in DMF (10 mL) is added dropwise and the reaction mixture is allowed to reach 25 °C during 2 h. At 0 °C, potassium tert-butoxide (3.6 g, 33.5 mmol, 1.1 eq) is then added portion wise and the reaction mixture is vigorously stirred for 16 h at 70 °C in a closed reactor. After addition of water (100 mL) the organic phase is separated and the aqueous layer is extracted with diethyl ether (3 × 50 mL). The combined organic layers are dried over sodium sulfate before being evaporated. The crude product was purified by chromatography on silica gel using ethyl acetate / cyclohexane (2 : 98) as eluent which afforded pure 3-(bromodifluoromethoxy)-2,6-dichloropyridine (**15**, 4.1 g, 17.7 mmol, 58 %) as a pale yellow oil.
¹H NMR (CDCl₃, 300 MHz): δ = 7.60 (d, *J* = 8.5 Hz, 1 H), 7.26 (d, *J* = 8.5 Hz, 1H). - ¹³C NMR (CDCl₃, 75 MHz): δ = 148.1, 144.4, 143.2, 133.4, 124.3, 114.7 (t, *J* = 113 Hz). - MS(EI): m/z = 292 [M⁺], 212 [M⁺-Br].

### 2,6-Dichloro-3-trifluoromethoxypyridine (4)

3-(Bromodifluoromethoxy)-2,6-dichloropyridine (**15**, 4.0 g, 13.7 mmol) is added dropwise to a molten mixture of SbF₃ (2.5 g, 13.7 mmol, 1 eq) and SbCl₅ (1.4 g, 0.6 mL, 4.5 mmol, 0.33 eq) at 120 °C and stirred for 16 h at 140 °C. The mixture is then cooled to 0 °C and dissolved in dichloromethane (50 mL). The solution is washed with saturated aqueous solutions of sodium hydrogen carbonate and brine, the aqueous layer is extracted with dichloromethane (2 × 50 mL). The combined organic layers are dried over sodium sulfate before being evaporated. The crude product is distilled under vacuum to afford pure 2,6-dichloro-3-trifluoromethoxypyridine (**4**, 1.0 g, 4.3 mmol, 31 %) as a colorless oil; b.p. 65-66 °C / 20 mbar.
¹H NMR (CDCl₃, 300 MHz): δ = 7.62 (d, *J* = 8.5 Hz, 1 H), 7.26 (d, *J* = 8.5 Hz, 1 H). - ¹³C NMR (CDCl₃, 75 MHz): δ = 148.2, 144.5, 141.8, 133.3, 120.7 (q, *J* = 261 Hz), 124.4. - MS(EI): m/z = 231 [M⁺], 195 [M⁺-Cl].

### 5-(Bromodifluoromethoxy)-2-chloropyridine (16)

At 0 °C, sodium hydride (60%, 1.5 g, 46.3 mmol, 1.5 eq) is added portion wise to a solution of 2-chloro-5-hydroxypyridine (4.0 g, 30.8 mmol) in DMF (50 mL). The reaction mixture is then vigorously stirred for 1 h at 25 °C and 30 min at 55°C. At 0 °C, a solution of dibromodifluoromethane (39.0 g, 17.0 mL, 185 mmol, 4 eq) in DMF (10 mL) is added dropwise and the reaction mixture is allowed to reach 25 °C during 2 h. At 0 °C, potassium *tert*-butoxide (3.5 g, 33.8 mmol, 1.1 eq) is then added portion wise and the reaction mixture is vigorously stirred for 16 h at 70 °C in a closed reactor. After addition of water (100 mL) the organic phase is separated and the aqueous layer is extracted with diethyl ether (3 × 50 mL). The combined organic layers are dried over sodium sulfate before being evaporated. The crude product is purified by chromatography on silica gel using ethyl acetate / cyclohexane (2 : 98) as eluent which affords pure 5-(bromodifluoromethoxy)-2-chloropyridine (**16**, 1.7 g, 6.8 mmol, 22 %) as a pale yellow oil.
¹H NMR (CDCl₃, 300 MHz): δ = 8.27 (d, *J* = 2.9 Hz, 1H), 7.48 (dd, *J* = 8.7, 2.9 Hz, 1 H), 7.32 (d, *J* = 8.7 Hz, 1 H). -¹³C NMR (CDCl₃, 75 MHz): δ = 149.4, 146.5, 143.1, 131.8, 125.1, 114.1 (t *J* = 311 Hz ). - MS(EI): m/z = 258 [M⁺], 178 [M⁺-Cl].

### 2-Chloro-5-trifluoromethoxypyridine (6)

5-(Bromodifluoromethoxy)-2-chloropyridine (**16**, 2.0 g, 10.1 mmol) is added dropwise to a molten mixture of SbF₃ (2.77 g, 15.0 mmol, 1.5 eq) and SbCl₅ (1.5 g, 0.65 mL, 5.0 mmol, 0.5 eq) at 120 °C and stirred for 16 h at 140 °C. The mixture is then cooled to 0 °C and dissolved in dichloromethane (100 mL). The solution is washed with saturated aqueous solutions of sodium hydrogen carbonate and brine, the aqueous layer is extracted with dichloromethane (2 × 50 mL). The combined organic layers are dried over sodium sulfate before being evaporated. The crude product is distilled under vacuum to afford pure 2-chloro-5-trifluoromethoxypyridine (**6**, 0.5 g, 2.5 mmol, 25%) as a colorless oil; b.p. 41-43 °C / 20 mbar.
¹H NMR (CDCl₃, 300 MHz): δ = 8.35 (s, 1 H), 7.55 (d, *J* = 8.7 Hz, 1H), 7.41 (d, *J* = 8.7 Hz, 1 H). -1³C NMR (CDCl₃, 75 MHz): δ = 149.3, 142.7, 140.1, 131.3, 120.2 (q, *J* = 259 Hz), 125.1. -MS(EI): m/z = 197 [M⁺], 162 [M⁺-Cl].

### Comparative example 3:

The formation of chlorothionoformates fails with non-halogenated hydroxypyridines:

Attempted conversion of non-chlorinated hydroxypyridines into chlorothionoformates

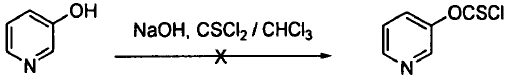

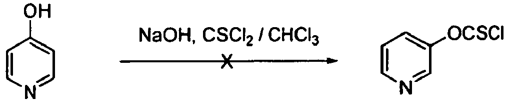

### Comparative example 4:

One aspect of the process according to the invention is the use of halogen, preferably chlorine atoms in the alpha-position of the pyridine ring. The comparative example 4 shows, that no influence on reactivity and yield can be observed as long as 2-, 6- or 2,6-dichloro-substituted pyridines are used. When the Cl-atoms are moved to the 3-, 4- or 5-position, respectively, the trichloromethoxylation (steps A and B) affords compounds of fomula II with smaller yields asside with the formation of dimerization products. However, the trifluoromethoxylation according to step (C) fails completely. Only traces of chlorodifluoromethoxy-substituted compounds are obtained.

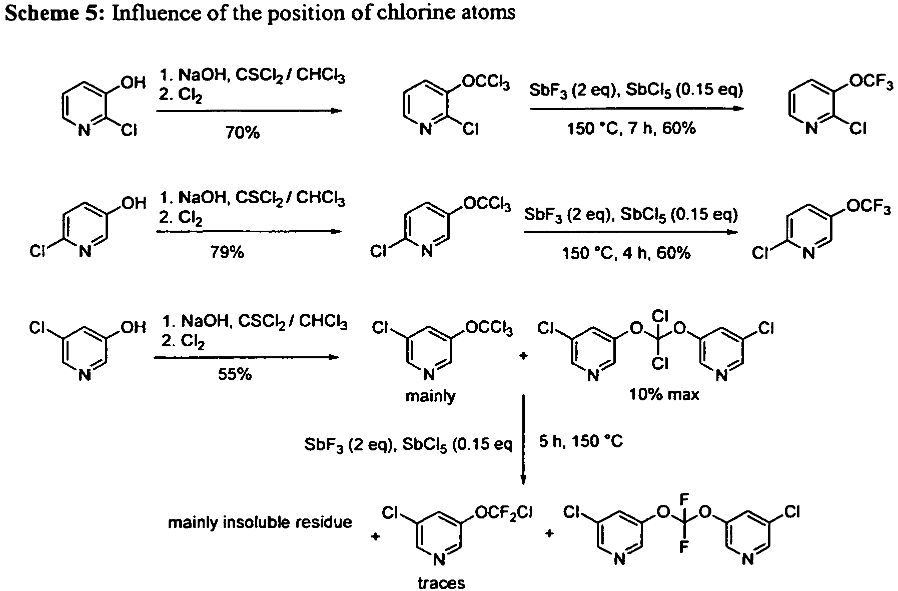

### Comparative example 5:

A further aspect of the invention is the use of chlorine atoms in the □-position of the pyridine ring. Reactivity and yield of the process steps (A) and (B) are not affected when the Cl-atoms are replaced by Br- or F-atoms. However, the process step (C) works only well with Cl-atoms In the case of Br-atoms mixtures of OCF₂Cl- and OCF₃- products are formed. In case of F-atoms mixtures of OCF₂Cl- and OCF₃- and OCFCl₂-products are obtained. 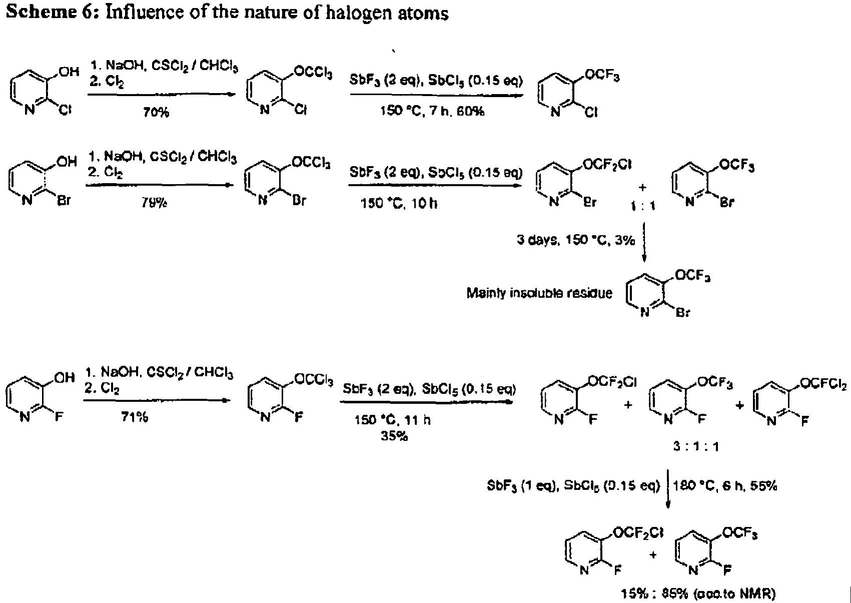 Examples for (chlorodifluoromethoxy)pyridines -Comparative Examples-

### 2-Methyl-5-(chlorodifluoromethoxy)pyridine

### 2-Methyl-5-trichloromethoxypyridine

At 0 °C, thiophosgene (5.7 g, 3.9 mL, 48.0 mmol, 1 eq) in chloroform (30 mL) was added dropwise to a solution of 3-chloro-5-hydroxypyridine (5.0 g, 48.0 mmol) in aqueous sodium hydroxide (5%, 41 mL). The reaction mixture was vigorously stirred for 2 h at 0 °C before being extracted with chloroform (3 × 50 mL). The combined organic layers were washed with diluted hydrochloric acid (1 N, 20 mL) and water (20 mL), dried over sodium sulfate before being filtrated. At 25 °C, the filtrate was then saturated with chlorine until the reaction mixture began to warm up. After 2 h at 25 °C, another excess of chlorine was added until a yellow solution was obtained. After 24 h at 25 °C, excess of chlorine was removed by a stream of Ar gas and the solution was evaporated. The crude oil was distilled under vacuum to afford pure 2-methyl-5-trichloromethoxypyridine (8.6 g, 38.4 mmol, 80%) as colorless crystals; m.p. 131-133 °C.
1H NMR (CDCl3, 300 MHz): δ = 8.56 (d, J = 2.6 Hz, 1H), 8.24 (dd, J = 8.8, 2.6 Hz, 1H), 7.77 (d, J = 8.8 Hz, 1H), 2.89 (s, 3 H). - 13C NMR (CDCl3, 75 MHz): δ = 153.1, 148.1, 139.5, 135.1, 128.9, 111.9, 19.3. - MS(EI): m/z = 224 [M+], 189 [M+-Cl].

### 2-Methyl-5-(chlorodifluoromethoxy)pyridine

2-Methyl-5-trichloromethoxypyridine (6.0 g. 27.0 mmol) was added dropwise to a molten mixture of SbF3 (4.7 g, 27.0 mmol, 1.0 eq) and SbCl5 (0.8 g, 0.3 mL, 2.7 mmol, 0.1 eq) at 120 °C and stirred for 7 h at 150 °C. GC monitoring indicated 100% conversion. The mixture was then cooled to 0 °C and dissolved in dichloromethane (200 mL). The solution was neutralized with saturated aqueous solution of sodium hydrogencarbonate (200 mL) and potassium fluoride (20%, 100 mL), the aqueous layer was extracted with dichloromethane (2 × 100 mL). The combined organic layers were dried over sodium sulfate and the solvent was distilled. The crude product was distilled under vacuum to afford pure 2-methyl-5-trifluoromethoxypyridine (2.1 g, 10.8 mmol, 40%) as a colorless oil; b.p. 59-62 °C / 17 mbar.
¹H NMR (CDCl3, 300 MHz): δ = 8.35 (d, J = 2.6 Hz, 1 H), 7.37 (dd, J = 8.5, 2.6 Hz, 1 H), 7.10 (d, J = 8.5, 1H), 2.49 (s, 3 H). - 19F NMR (CDCl3, 282 MHz): δ - -27.0. -13C NMR (CDCl3, 75 MHz): δ = 157.3, 145.0, 142.6, 129.3, 125.3 (t, J=290 Hz), 123.7, 23.8.

### Examples of Functionalization -not according to the invention-

### 1. Functionalized 3-OCF₃-pyridine;

### 2-Chloro-5-trinfloromethoxy isonicotinic acid (17)

At 0 °C, diisopropylamine (0.25 g, 0.35 mL, 2.5 mmol, 1 eq) was added dropwise to a solution of butyllithium (1.56 M in hexane, 1.6 mL, 2.5 mmol, 1 eq) in THF (4 mL). At -78 °C, a solution of 2-chloro-5-trifluoromethoxypyridine (6, 0.5 g, 2.5 mmol, I eq) in THF (2 mL) was added dropwise and the reaction mixture was stirred for 2 h at this temperature. Then, the mixture was poured onto an excess of freshly crushed dry ice and allowed to reach 25 °C before being treated with an aqueous solution of sodium hydroxide (5%, 5 mL). The resulting aqueous layer was collected, washed with diethylether (2 mL) and acidified to pH 4 by dropwise addition of hydrochloric acid (6 N, 1 mL) before being extracted with ethyl acetate (3 × 3 mL). The combined organic layers were dried over sodium sulfate and the solvents evaporated to afford pure 2-chloro-5-trifluoromethoxy isonicotinic acid (**17**, 0.46 g, 1.9 mmol, 75%) as a white powder; m.p. 150-152 °C.
¹H NMR (CDCl₃, 300 MHz): δ = 8.45 (s, 1H), 7.84 (s, 1 H), 3.88 (br s, 1H). -¹⁹F NMR (CDCl₃, 282 MHz): δ = -57.8. -¹³C NMR (CDCl₃, 75 MHz): δ = 150.3, 144.9, 142.5, 137.1, 133.8, 126.1, 120.3 (q, *J* = 260 Hz). - MS(APCI+): m/z = 241 [M⁺], 197 [M⁺-CO₂].

### 3-Trifluoromethoxy isonicotinic acid (18)

At 25 °C, palladium (10% on charcoal, 200 mg) was added with stirring to a solution of 2-chloro-5-trifluoromethoxy isonicotinic acid (**17**, 660 mg, 2.7 mmol) and ammonium formate (290 mg, 4.6 mmol, 1.7 eq) in methanol (5 mL). The reaction mixture was stirred for 16 h at 55 °C before being filtrated under suction and the filtrate evaporated. The residue was partitioned between ethyl acetate (2× 10 mL) and 2.0 M hydrochloric acid (15 mL). The combined organic layers were dried over sodium sulfate before being evaporated to afford pure 3-trifluoromethoxy isonicotinic acid (**18**, 450 mg, 3.0 mmol, 81%) as a white powder; m.p. 181-183 °C.
¹H NMR (CD₃OD, 300 MHz): δ = 8.42 (d, *J =* 4.9 Hz, 1H), 8.41 (s, 1 H), 7.46 (d, *J* = 4.9 Hz, 1H). -¹⁹F NMR ((CD₃)₂CO, 282 MHz): δ = -59.0. -¹³C NMR (CD₃OD, 75 MHz): δ = 164.0, 148.7, 144.5, 143.0, 134.0, 124.7, 120.4 (q, *J* = 258 Hz). - HRMS (ESI negative) for C₇H₃F₃NO₃ [M-H]: calcd. 206.0060; found 206.0033.

### 2-Chloro-3-trifluoromethoxy isonicotinic acid (19)

At 0 °C, diisopropylamine (1.2 g, 1.6 mL, 11.1 mmol, 1.1 eq) was added dropwise to a solution of butyllithium (1.56 M in hexane, 7.1 mL, 11.1 mmol, 1.1 eq) in THF (15 mL). At -78 °C, a solution of 2-chloro-3-trifluoromethoxy pyridine (**8**, 2.0 g, 10.1 mmol, 1 eq) in THF (5 mL) was added dropwise and the reaction mixture was stirred for 2 h at this temperature. The mixture was then poured onto an excess of freshly crushed dry ice before being treated with an aqueous solution of sodium hydroxide (5%, 20 mL). The resulting aqueous layer was collected, washed with diethylether (10 mL) and acidified to pH 4 by dropwise addition of hydrochloric acid (6 N, 4 mL). After extraction with ethyl acetate (3 × 15 mL), the combined organic layers were dried over sodium sulfate before being evaporated to afford pure 2-chloro-3-trifluoromethoxy isonicotinic acid (**18**, 1.7 g, 7.0 mmol, 70%) as a white powder; m.p. 157-159 °C.
¹H NMR (CD₃OD, 300 MHz): δ = 8.41 (d, *J* = 4.9 Hz, 1 H), 7.70 (d, *J* = 4.9 Hz, 1 H). - ¹⁹F NMR (CDCl₃, 282 MHz): δ = -57.3. - ¹³C NMR (CD₃OD, 75 MHz): δ = 163.8, 148.4, 146.7, 138.9, 137.8, 123.9, 120.3 (q, *J* = 260 Hz). - HRMS (APCI positive) for C₆H₃ClF₃NO [M-CO₂]: calcd. 197.9928; found 197.9956. - C₇H₃ClF₃NO₃ (241): calcd. (%) C 34.81, H 1.25, N 5.80; found C 34.38, H 1.46, N 5.83.

### 4-Amino-2-chloro-3-trifluoromethoxy pyridine (20)

At 0 °C, diisopropylamine (1.2 g, 1.6 mL, 11.1 mmol, 1.1 eq) was added dropwise to a solution of butyllithium (1.56 M in hexane, 7.1 mL, 11.1 mmol, 1.1 eq) in THF (15 mL). At -78 °C, a solution of 2-chloro-3-trifluoromethoxy pyridine (8, 2.0 g, 10.1 mmol, 1 eq) in THF (5 mL) was added dropwise followed after 2 h by benzenesulfonyl azide (2.8 g, 15.2 mmol, 1.5 eq). The reaction mixture was allowed to reach 25 °C before being treated with a saturated aqueous solution of ammonium chloride (20 mL) and extracted with diethylehter (3 × 10 mL). The combined organic layers were dried over sodium sulfate and evaporated to afford a crude red oil of 4-azido-2-chloro-3-trifluoromethoxypyridine. It was then dissolved in anhydrous diethylether (90 mL) and added dropwise to a suspension of lithium aluminium hydride (430 mg, 11.2 mmol, 1.1 eq) in diehtylether (90 mL). The reaction mixture was heated under reflux for 5 h before being treated with water (100 mL) and extracted with diethylether (3 × 30 mL). The combined organic layers were dried over sodium sulfate before being evaporated. The crude product was purified by chromatography on silica gel using ethyl acetate/cyclohexane (1:4) as eluent which afforded pure 4-amino-2-chloro-3-trifluoromethoxy pyridine (**20**, 1.6 g, 7.5 mmol, 75%) as yellow crystals; m.p. 54-56 °C.
¹H NMR (CDCl₃, 300 MHz): δ = 7.78 (d, *J* = 5.5 Hz, 1 H), 6.51 (d, *J =* 5.5 Hz, 1 H), 4.69 (bs, 2 H). -¹⁹F NMR (CDCl₃, 282 MHz): δ = -57.0. -¹³C NMR (CDCl₃, 75 MHz): δ = 171.2, 148.4, 147.0, 146.3, 120.9 (q, *J* = 260 Hz), 111.0. - HRMS (ESI positive) for C₆H₅ClF₃N₂O [M+H]: calcd. 213.0037; found 213.0051. - C₇H₃ClF₃NO₃ (212): calcd. (%) C 33.90, H 1.90, N 13.18; found C 33.45, H 2.10, N 13.40.

### 4-Amino-3-trifluoromethoxy pyridine (21)

At 25 °C, palladium (10% on charcoal, 470 mg) was added with stirring to a solution of 4-amino-2-chloro-3-trifluoromethoxypyridine (**20**, 1.5 g, 7.0 mmol) and ammonium formate (880 mg, 14.0 mmol, 2 eq) in methanol (12 mL). The reaction mixture was stirred for 16 h at 55 °C before being filtrated under suction and the solvent evaporated. The residue was partitioned between ethyl acetate (2× 20 mL) and water (30 mL). The combined organic layers were dried over sodium sulfate before being evaporated. The crude yellow oil was distilled under vacuum to afford pure 4-amino-3-trifluoromethoxy pyridine (**21**, 1.0 g, 5.6 mmol, 80%) as a colorless oil; b.p 110-114 °C / 14 mbar.
¹H NMR (CDCl₃, 300 MHz): δ = 8.19 (s, 1 H), 8.05 (d, *J* = 5.5 Hz 1H), 6.59 (d, *J* = 5.5 Hz, 1 H), 4.41 (bs, 2 H). - ¹⁹F NMR (CDCl₃, 282 MHz): δ = -58.7. - ¹³C NMR (CD₃OD, 75 MHz): δ = 150.8, 148.5, 143.5, 132.9, 120.8 (q, *J* = 259 Hz), 110.4. - C₆H₅F₃N₂O (178): calcd. (%) C 40.46, H 2.83, N 15.73; found C 40.28, H 2.74, N 15.94.

### 2-Chloro-4-iodo-3-trifluoromethoxy pyridine (22)

At 0 °C, diisopropylamine (0.6 g, 0.8 mL, 5.5 mmol, 1.1 eq) was added dropwise to a solution of butyllithium (1.56 M in hexane, 3.5 mL, 5.5 mmol, 1.1 eq) in THF (8 mL). At -78 °C, a solution of 2-chloro-3-trifluoromethoxypyridine (**8**, 1.0 g, 5.0 mmol, 1 eq) in THF (3 mL) was added dropwise followed after 2 h by a solution of iodide (1.3 g, 7.5 mmol, 1.5 eq) in THF (5 mL). The reaction mixture was allowed to reach 25 °C before being treated with a saturated aqueous solution of sodium sulfite (10 mL) and extracted with dichloromethane (3 × 10 mL). The combined organic layers were dried over sodium sulfate before being evaporated. The crude dark oil was distilled under vacuum (b.p. 78-81 °C / 16 mbar) to afford pure 2-chloro-4-iodo-3-trifluoromethoxy pyridine (**22**, 1.0 g, 3.0 mmol, 62%) as colorless needles; m.p. 40-43 °C.
¹H NMR (CDCl₃, 300 MHz): δ = 7.88 (d, *J* = 5.0 Hz, 1 H), 7.69 (d, *J =* 5.0 Hz, 1 H). - ¹⁹F NMR (CDCl₃, 282 MHz): δ = -58.2. - ¹³C NMR (CDCl₃, 75 MHz): δ = 153.4, 147.7, 145.7, 144.1, 134.4, 120.7 (q, *J* = 260 Hz).

### 2-Bromo-3-trifluoromethoxypyridine (23)

A solution of 2-chloro-3-trifluoromethoxypyridine (**8**, 4.0 g, 20.2 mmol) and bromotrimethylsilane (6.3 g, 5.4 mL, 40.4 mmol, 2 eq) in propionitrile (20 mL) was heated under reflux for 24 h. GC monitoring indicated 100% conversion. The mixture was distilled in vacuum to afford pure 2-bromo-3-trifluoromethoxypyridine (**23**, 3.9 g, 16.1 mmol, 80%) as a colorless oil; b.p. 63-67 °C / 13 mbar.
¹H NMR (CDCl₃, 300 MHz): δ = 8.38 (dd, *J* = 4.7, 1.6 Hz, 1 H), 7.68 (d, *J* = 8.1, 1.6 Hz, 1 H), 7.34 (dd, *J* = 8.1, 4.7 Hz, 1 H). - ¹⁹F NMR (CDCl₃, 282 MHz): δ = -58.0. - ¹³C NMR (CDCl₃, 75 MHz): δ = 147.8, 144.8, 142.2, 130.5, 123.7, 120.3 (q, *J* = 260 Hz). - C₆H₃ClF₃NO (241): calcd. (%) C 29.78, H 1.25, N 5.79; found C 29.96, H 1.41, N 5.64.

### 3-Trifluoromethoxy picolinic acid (24)

At -100 °C, butyllithium (1.56 M in hexane, 5.5 mL, 8.3 mmol, 1 eq) was added dropwise to a solution 2-bromo-3-trifluoromethoxy pyridine (**23**, 2.0 g, 8.3 mmol) in dried toluene (15 mL). After 2 h at -78 °C, the mixture was poured onto an excess of freshly crushed dry ice before being treated with an aqueous solution of sodium hydroxide (5%, 15 mL). The resulting aqueous layer was collected, washed with diethylether (10 mL) and acidified to pH 4 by dropwise addition of hydrochloric acid (6 N, 4 mL) before being extracted with ethyl acetate (3 × 10 mL). The combined organic layers were dried over sodium sulfate and evaporated to afford pure 3-trifluoromethoxy picolinic acid (**24**, 1.1 g, 5.3 mmol, 64%) as a white powder; m.p. 84-87 °C.
¹H NMR (CD₃OD, 300 MHz): δ = 8.54 (d, *J* = 4.5 Hz, 1 H), 7.87 (d, *J* = 8.5 Hz, 1 H), 7.63 (dd, *J* = 8.5, 4.5 Hz, 1 H). -¹⁹F NMR ((CD₃)₂CO, 282 MHz): δ = -58.7 - ¹³C NMR (CD₃OD, 75 MHz): δ = 164.5, 147.5, 144.5, 143.4, 131.2, 127.6, 120.3 (q, *J* = 260 Hz). - C₇H₄F₃NO₃ (207): calcd. (%) C 40.59, H 1.95, N 6.76; found C 40.21, H 2.17, N 6.97.

### 2-Chloro-5-trifluoromethoxy-4-trimethylsilyl pyridine (25)

At 0 °C, diisopropylamine (2.8 g, 3.9 mL, 27.8 mmol, 1.1 eq) was added dropwise to a solution of butyllithium (1.56 M in hexane, 17.8 mL, 27.8 mmol, 1.1 eq) in THF (40 mL). At -78 °C, a solution of 2-chloro-5-trifluoromethoxy pyridine (**6**, 5.0 g, 25.3 mmol, 1 eq) in THF (10 mL) was added dropwise and the reaction mixture was stirred for 2 h at this temperature. Chlorotrimethylsilane (3.0 g, 3.5 mL, 27.8 mmol, 1.1 eq) was then added and the mixture was allowed to reach 25 °C before being neutralized with water (40 mL) and extracted with diethylether (3 × 15 mL). The combined organic layers were dried over sodium sulfate before being evaporated. The crude product was distilled under vacuum to afford pure 2-chloro-5-trifluoromethoxy-4-trimethylsilyl pyridine (**25**, 6.3 g, 23.2 mmol, 92%) as a colorless oil; b.p. 96-98 °C / 14 mbar.
¹H NMR (CDCl₃, 300 MHz): δ = 8.20 (s, 1 H), 7.28 (s, 1 H), 0.26 (s, 9 H). -¹³C NMR (CDCl₃, 75 MHz): δ = 149.9, 148.5, 146.2, 139.5, 130.0, 120.3 (q, *J* = 260 Hz), -1.9. - MS(EI): m/z = 269 [M⁺].

### 6-Chloro-3-trifluoromethoxy picolinic acid (26)

At 0 °C, diisopropylamine (2.1 g, 2.9 mL, 20.5 mmol, 1.1 eq) was added dropwise to a solution of butyllithium (1.56 M in hexane, 13.2 mL, 20.5 mmol, 1.1 eq) in THF (30 mL). At -78 °C, a solution of 2-chloro-5-trifluoromethoxy-4-trimethylsilyl pyridine (**25**, 5.0 g, 18.6 mmol) in THF (10 mL) was added dropwise and the reaction mixture was stirred for 3 h at this temperature. Then, the mixture was poured onto an excess of freshly crushed dry ice and allowed to reach 25 °C before being treated with an aqueous solution of sodium hydroxide (5%, 30 mL). The resulting aqueous layer was collected, washed with diethylether (10 mL) and acidified to pH 4 by dropwise addition of hydrochloric acid (6 N, 10 mL) before being extracted with ethyl acetate (3 × 15 mL). The combined organic layers were dried over sodium sulfate and the solvents evaporated. Crystallization of the residue from chloroform afforded colorless needles of pure 6-chloro-3-trifluoromethoxy picolinic acid (**26**, 2.7 g, 11.3 mmol, 61 %); m.p. 93-96 °C.
¹H NMR (CDCl₃, 300 MHz): δ = 7.72 (dq, *J* = 8.7, 1.3 Hz, 1 H), 7.58 (d, *J* = 8.7 Hz, 1 H). - ¹⁹F NMR ((CDCl₃, 282 MHz): δ = -58.2 - ¹³C NMR (CDCl₃, 75 MHz): δ = 161.4, 148.1, 145.1, 139.6, 134.7, 130.0, 120.5 (q, *J* = 261 Hz).

### 2-Amino-3-trifluoromethoxypyridine (27)

2-Chloro-3-trifluoromethoxypyridine (**8**, 1.0 g, 5.0 mmol), benzophenone imine (1.1 g, 6.0 mmol, 1.2 eq), KOtBu (0.85 g, 7.5 mmol, 1.5 eq), DPEphos (0.03 g, 0.05mmol, 0.01 eq), Pd₂dba₃ (0.07 g, 0.1 mmol, 0.02 eq) were introduced in dry toluene (15 mL) and the reaction mixture was heated at 70 °C and vigorously stirred for 2 h. GC monitoring indicated 100% conversion. The mixture was allowed to cool to ambient temperature before being filtrated on celite and washed with ethyl acetate. The filtrate was poured onto a 10% aqueous solution of citric acid (20 mL) and the reaction mixture was then vigorously stirred for 16 h at room temperature. GC monitoring of the organic phase indicated disappearance of starting reagent and formation of diphenylketone. The aqueous phase was adjusted to pH 9-10 with 5% sodium hydroxide (30 mL) and extracted with ethyl acetate (5 × 20 mL). The combined organic layers were dried over sodium sulfate before being evaporated to afford a crude brown powder. Crystallization in cyclohexane provided pure 2-amino-3-trifluoromethoxy pyridine (**27**, 0.40 g, 2.2 mmol, 44%) as colorless needles; m.p. 69-71 °C.
¹H NMR (CDCl₃, 300 MHz): δ = 8.00 (dd, *J* =4.9, 1.5 Hz, 1 H), 7.39 (dd, *J=* 8.0, 1.5 Hz, 1 H), 6.68 (dd, *J* = 8.0, 4.9 Hz, 1 H), 4.84 (bs, 2 H). - ¹⁹F NMR (CDCl₃, 282 MHz): δ = -58.7. - ¹³C NMR (CDCl₃, 75 MHz): δ = 151.8, 146.1, 133.1, 128.3, 120.8 (q, *J* = 259 Hz), 113.8.

### 2-Chloro-4-iodo-5-trifluoromethoxypyridine (28)

At 0 °C, diisopropylamine (2.2 g, 3.1 mL, 22.2 mmol, 1.1 eq) was added dropwise to a solution of butyllithium (1.56 M in hexane, 14.2 mL, 22.2 mmol, 1.1 eq) in THF (35 mL). At -78 °C, a solution of 2-chloro-5-trifluoromethoxypyridine (**6**, 4.0 g, 20.2 mmol, 1 eq) in THF (10 mL) was added dropwise followed after 2 h by a solution of iodine (5.7 g, 22.2 mmol, 1.1 eq) in THF (10 mL). The reaction mixture was allowed to reach 25 °C before being treated with a saturated aqueous solution of sodium sulfite (30 mL) and extracted with dichloromethane (3 × 20 mL). The combined organic layers were dried over sodium sulfate before being evaporated. The crude dark oil was distilled under vacuum (b.p. 95-97 °C / 16 mbar) to afford pure 2-chloro-4-iodo-5-trifluoromethoxypyridine (**28**, 5.8 g, 18.0 mmol, 89%) as colorless needles; m.p. 85-87 °C.
¹H NMR (CDCl₃, 300 MHz): δ = 8.17 (s, 1 H), 7.80 (s, 1 H). - ¹⁹F NMR (CDCl₃, 282 MHz): δ = -58.0. - ¹³C NMR (CDCl₃, 75 MHz): δ = 149.4, 146.6, 141.3, 134.9, 120.5 (q, *J* = 260 Hz), 103.5.

### 2-Chloro-6-iodo-5-trifluoromethoxypyridine (29)

At -78 °C, a solution of LDA: diisopropylamine (0.6 g, 0.9 mL, 6.2 mmol, 2 eq) and butyllithium (1.56 M in hexane, 4.0 mL, 6.2 mmol, 1.1 eq) in THF, (6 mL) was added dropwise to a solution of 2-chloro-4-iodo-5-trifluoromethoxypyridine (**28**, 1.0 g, 3.1 mmol) in THF (6 mL) and the reaction mixture was stirred for 1 h at this temperature. Then, the mixture was hydrolyzed with an aqueous solution of hydrochloric acid (5%, 5 mL) before being extracted with ethyl acetate (3 × 5 mL). The combined organic layers were dried over sodium sulfate and the solvents evaporated. The crude oil was purified by distillation under vacuum (b.p. 96-100 °C / 16 mbar) to afford a mixture (0.5 g, 1.55 mmol, 50%) of 2-chloro-4-iodo-5-trifluoromethoxypyridine (**28**) and 2-chloro-6-iodo-5-trifluoromethoxypyridine (**29**) in a 2 : 8 ratio as a pale yellow oil. At -78 °C, the mixture was dissolved in THF (4 mL) and BuLi (1.56M in hexane, 0.2 mL, 0.31 mmol, 0.2 eq) was added dropwise. After 10 min at this temperature, it was neutralized with an aqueous solution of hydrochloric acid (5%, 5 mL) before being extracted with diethyl ether (3 × 5 mL). The combined organic layers were dried over sodium sulfate and the solvents evaporated. The crude oil was purified by distillation under vacuum (b.p. 98-102 °C / 16 mbar) to afford pure 2-chloro-6-iodo-5-trifluoromethoxypyridine (**29**, 0.3 g, 0.93 mmol, 60% comparatively to the previous mixture, 30% overall yield) as a pale yellow oil.
¹H NMR (CDCl₃, 300 MHz): δ = 7.47 (d, *J* = 8.4 Hz, 1 H), 7.35 (d, *J* = 8.4 Hz, 1 H). - ¹⁹F NMR (CDCl₃, 282 MHz): δ = -57.8. - ¹³C NMR (CDCl₃, 75 MHz): δ = 147.2, 145.6, 128.9, 124.2, 120.3 (q, *J* = 260 Hz), 111.4.

### 2,6-Dichloro-4-iodo-5-tritluoromethoxypyridine (30)

At 0 °C, diisopropylamine (1.3 g, 1.9 mL, 13.3 mmol, 1.1 eq) was added dropwise to a solution of butyllithium (1.56 M in hexane, 8.5 mL, 13.3 mmol, 1.1 eq) in THF (20 mL). At -78 °C, a solution of 2,6-dichloro-3-trifluoromethoxypyridine (**4**, 2.8 g, 12.1 mmol) in THF (10 mL) was added dropwise followed after 2 h by a solution of iodine (3.3 g, 13.3 mmol, 1.1 eq) in THF (10 mL). The reaction mixture was allowed to reach 25 °C before being treated with a saturated aqueous solution of sodium sulfite (20 mL) and extracted with dichloromethane (3 × 10 mL). The combined organic layers were dried over sodium sulfate before being evaporated. The crude dark oil was distilled under vacuum (b.p. 78-80 °C / 1 mbar) to afford pure 2,6-dichloro-4-iodo-5-trifluoromethoxypyridine (**30**, 2.6 g, 7.3 mmol, 60%) as a colorless oil which crystallized on standing.
¹H NMR (CDCl₃, 300 MHz): δ = 8.73 (s, 1 H). -¹⁹F NMR (CDCl₃, 282 MHz): δ = - 54.9. -¹³C NMR (CDCl₃, 75 MHz): δ = 148.4, 144.4, 143.4, 134.1, 120.5 (q, *J* = 260 Hz), 106.0.

### 2-Bromo-5-tritluoromethoxy pyridine (31)

A solution of 2-chloro-5-trifluoromethoxy pyridine (**6**, 7.0 g, 35.4 mmol) and bromotrimethylsilane (10.8 g, 9.3 mL, 70.8 mmol, 2 eq) in propionitrile (35 mL) was heated under reflux for 24 h. GC monitoring indicated 100% conversion. The mixture was distilled in vacuum to afford pure 2-bromo-4-trifluoromethoxy pyridine (**31**, 7.0 g, 28.7 mmol, 81 %) as a colorless oil; b.p. 63-66 °C / 14 mbar.
¹H NMR (CDCl₃, 300 MHz): δ = 8.34 (d, *J* = 2.8 Hz, 1 H), 7.56 (d, *J* = 8.7 Hz, 1H), 7.45 (dd, *J* = 8.7, 2.8 Hz, 1 H). - ¹⁹F NMR (CDCl₃, 282 MHz): δ = -58.8. - ¹³C NMR (CDCl₃, 75 MHz): δ = 145.6, 143.1, 139.2, 131.4, 129.0, 120.1 (q, *J* = 260 Hz). - C₆H₃BrF₃NO (241): calcd. (%) C 29.78, H 1.25, N 5.79; found C 29.96, H 1.41, N 5.64.

### 5-Trifluoromethoxy picolinic acid (32)

At -100 °C, butyllithium (1.56 M in hexane, 5.5 mL, 8.3 mmol, 1 eq) was added dropwise to a solution 2-bromo-5-trifluoromethoxy pyridine (**31**, 2.0 g, 8.3 mmol) in dried toluene (15 mL). After 2 h at -78 °C, the mixture was poured onto an excess of freshly crushed dry ice before being treated with an aqueous solution of sodium hydroxide (5%, 15 mL). The resulting aqueous layer was collected, washed with diethylether (10 mL) and acidified to pH 4 by dropwise addition of hydrochloric acid (6 N, 4 mL) before being extracted with ethyl acetate (3 × 10 mL). The combined organic layers were dried over sodium sulfate and evaporated to afford pure 5-trifluoromethoxy picolinic acid (**32**, 1.0 g, 4.9 mmol, 60%) as a white powder; m.p. 123-125 °C.
¹H NMR (CD₃OD, 300 MHz): δ = 8.56 (d, *J* = 1.2 Hz, 1 H), 8.19 (d, *J* = 8.6 Hz, 1 H), 7.87 (dd, *J* = 8.6, 1.2 Hz, 1 H). - ¹⁹F NMR (CDCl₃, 282 MHz): δ = -59.3. - ¹³C NMR (CD₃OD, 75 MHz): δ = 165.0, 148.3, 146.5, 141.8, 129.2, 126.3, 120.3 (q, *J* = 259 Hz). - C₇H₄F₃NO₃ (207): calcd. (%) C 40.59, H 1.95, N 6.76; found C 40.34, H 2.21, N 6.71.

### 2-Iodo-5-trifluoromethoxypyridine (33)

A suspension of 2-chloro-5-trifluoromethoxypyridine (**6**, 9.3 g, 47 mmol), chlorotrimethylsilane (5.1 g, 6.1 mL, 47 mmol, 1 eq) and sodium iodine (21.3 g, 142 mmol, 3 eq) in propionitrile (35 mL) was heated under reflux for 24 h. GC monitoring indicated 100% conversion. The reaction mixture was neutralized by addition of distilled water (100 mL) before being extracted with diethyl ether (3 × 40 mL). The combined organic layers were washed with a saturated aqueous solution of sodium sulfite (30 mL), dried over sodium sulfate before being evaporated. The crude dark oil was distilled under vacuum (90-93 °C / 26 mbar) to afford pure 2-iodo-4-trifluoromethoxypyridine (**33**, 6.7 g, 23.2 mmol, 4.9%) as a colorless oil which crystallized on standing.
¹H NMR (CDCl₃, 300 MHz): δ = 8.25 (d, *J* = 2.6 Hz, 1 H), 7.69 (d, *J* = 8.6 Hz, 1 H), 7.15 (dd, *J* = 8.6, 2.6 Hz, 1 H). - ¹⁹F NMR (CDCl₃, 282 MHz): δ = -58.9. - ¹³C NMR (CDCl₃, 75 MHz): δ = 146.3, 143.8, 135.7, 130.3, 120.1 (q, *J* = 259 Hz), 114.2.

### 2-Amino-5-trifluoromethoxy pyridine (34)

2-Chloro-5-trifluoromethoxypyridine (**6**, 1.0 g, 5.0 mmol), benzophenone imine (1.09 g, 6.0 mmol, 1.2 eq), NaOtBu (0.72 g, 7.5 mmol, 1.5 eq), DPEphos (0.03 g, 0.05mmol, 0.01 eq), Pd₂dba₃ (0.07 g, 0.1 mmol, 0.02 eq) were introduced in dry toluene (15 mL) and the reaction mixture was heated at 80 °C and vigorously stirred for 2 h. GC monitoring indicated 100% conversion. The mixture was allowed to cool to ambient temperature before being filtrated on celite and washed with ethyl acetate. The filtrate was poured onto a 10% aqueous solution of citric acid (30 mL) and the reaction mixture was then vigorously stirred for 16 h at room temperature. GC of the organic phase indicated disappearance of starting reagent and formation of diphenylketone. The aqueous phase was adjusted to pH 9-10 with 5% sodium hydroxide (40 mL) and extracted with ethyl acetate (5 × 20 mL). The combined organic layers were dried over sodium sulfate before being evaporated to afford a crude yellow powder. Crystallization in hexane provided pure 2-amino-5-trifluoromethoxy pyridine (**34**, 0.36 g, 2.0 mmol, 40%) as colorless needles; m.p. 71-73 °C.
¹H NMR (CDCl₃, 300 MHz): δ = 7.91 (d, *J =* 2.7 Hz, 1 H), 7.23 (dd, *J =* 2.7, 8.9 Hz, 1 H), 6.39 (d, *J* = 8.9 Hz, 1 H), 4.49 (bs, 2 H). - ¹⁹F NMR (CDCl₃, 282 MHz): δ = -58.9 - ¹³C NMR (CDCl₃, 75 MHz): δ = 157.1, 141.4, 138.7, 131.6, 120.6 (q, *J* = 256 Hz), 108.7. - C₆H₅F₃N₂O (178): calcd. (%) C 40.46, H 2.83, N 15.73; found C 40.46, H 2.90, N 16.02.

### 2-Chloro-3-trifluorometboxy-4-trimethylsilylpyridine (35)

At 0 °C, diisopropylamine (0.6 g, 0.8 mL, 5.6 mmol, 1.1 eq) was added dropwise to a solution of butyllithium (1.14 M in hexane, 4.9 mL, 5.6 mmol, 1.1 eq) in THF (10 mL). At -78 °C, a solution of 2-chloro-3-trifluoromethoxypyridine (**8**, 1.0 g, 5.1 mmol, 1 eq) in THF (4 mL) was added dropwise and the reaction mixture was stirred for 2 h at this temperature. Chlorotrimethylsilane (0.6 g, 0.7 mL, 5.6 mmol, 1.1 eq) was then added and the mixture was allowed to reach 25 °C before being neutralized with water (15 mL) and extracted with diethylether (3 × 10 mL). The combined organic layers were dried over sodium sulfate before being evaporated. The crude product was distilled under vacuum to afford pure 2-chloro-3-trifluoromethoxy-4-trimethylsilylpyridine (**35**, 950 mg, 3.5 mmol, 69%) as a colorless oil; b.p. 93-96 °C / 16 mbar.
¹H NMR (CDCl₃, 300 MHz): δ = 8.32 (d, *J* = 4.7 Hz, 1 H), 7.36 (d, *J* = 4.7 Hz, 1 H), 0.39 (s, 9 H). - ¹³C NMR (CDCl₃, 75 MHz): δ = 149.7, 147.9, 146.8, 145.9, 128.8, 120.5 (q, *J* = 260 Hz), -1.1.

### 6-chloro-5-tritluoromethoxy picolinic acid (36)

At 0 °C, 2,2,6,6-tetramethylpiperidine (0.3 g, 0.35 mL, 2.0 mmol, 1.1 eq) was added dropwise to a solution of butyllithium (1.54 M in hexane, 1.3 mL, 2.0 mmol, 1.1 eq) in THF (3 mL). At -78 °C, a solution 2-chloro-3-trifluoromethoxy-4-trimethylsilyl pyridine (**35**, 500 mg, 1.9 mmol, 1 eq) in THF (1 mL) was added dropwise and the reaction mixture was stirred for 2 h at this temperature. The mixture was then poured onto an excess of freshly crushed dry ice before being treated with an aqueous solution of sodium hydroxide (5%, 4 mL). The resulting aqueous layer was collected, washed with diethylether (3 mL) and acidified to pH 4 by dropwise addition of hydrochloric acid (6 N, 2 mL). After extraction with ethyl acetate (3 × 3 mL), the combined organic layers were dried over sodium sulfate before being evaporated. The crude oil was treated with tetrabutylammonium fluoride (1M in THF, 2.0 mL, 2.0 mmol, 1.1 eq) for 20 h at 25 °C. The mixture was neutralized by addition of hydrochloric acid (2N, 4 mL) and extracted with ethyl acetate (3 × 3 mL). The combined organic layers were dried over sodium sulfate before being evaporated to afford 6-chloro-5-trifluoromethoxy picolinic acid (**36**, 310 mg, 1.27 mmol, 67%) as a white powder; m.p. 99-102 °C.
¹H NMR (CDCl₃, 300 MHz): δ = 8.13 (d, *J* = 8.3 Hz, 1 H), 7.74 (dq, *J* = 8.3, 1.4 Hz, 1 H). - ¹⁹F NMR (CDCl₃, 282 MHz): δ = -58.1 - ¹³C NMR (CDCl₃, 75 MHz): δ = 163.4, 145.5, 144.0, 143.9, 130.8, 124.7, 120.2 (q, *J* = 263 Hz). - C₇H₃ClF₃NO₃ (241): calcd. (%) C 34.81, H 1.25, N 5.80; found C 34.56, H 1.49, N 5.47.

### Functionalized 2-OCF₃-pyridines

### 6-Chloro-2-trifluoromethoxy nicotinic acid (37)

At 0 °C, diisopropylamine (1.3 g, 1.8 mL, 12.6 mmol, 1 eq) was added dropwise to a solution of butyllithium (1.56 M in hexane, 8.3 mL, 12.6 mmol, 1 eq) in THF (25 mL). At -100 °C, a solution of 2-chloro-6-trifluoromethoxypyridine (**2**, 2.5 g, 12.6 mmol, 1 eq) in THF (5 mL) was added dropwise and the reaction mixture was stirred for 4 h at - 85 °C. The mixture was then poured onto an excess of freshly crushed dry ice before being treated with an aqueous solution of sodium hydroxide (5%, 25 mL). The resulting aqueous layer was collected, washed with diethylether (15 mL) and acidified to pH 4 by dropwise addition of hydrochloric acid (6 N, 8 mL). After extraction with ethyl acetate (3 × 20 mL), the combined organic layers were dried over sodium sulfate before being evaporated to afford pure 6-chloro-2-trifluoromethoxy nicotinic acid (**37**, 1.9 g, 7.9 mmol, 69%) as a white powder; m.p. 139-142 °C.
¹H NMR (CDCl₃, 300 MHz): δ = 8.32 (d, *J* = 8.2 Hz, 1 H), 7.29 (d, *J* = 8.2 Hz, 1 H). - ¹⁹F NMR (CDCl₃, 282 MHz): δ = -56.5 - ¹³C NMR (CDCl₃, 75 MHz): δ = 167.5, 154.2, 153.4, 145.3, 122.1, 119.3 (q, *J* = 260 Hz), 113.5. - C₇H₃ClF₃NO₃ (241): calcd. (%) C 34.81, H 1.25, N 5.80; found C 34.88, H 1.32, N 5.78.

### 2-Trifluoromethoxy nicotinic acid (38)

At 25 °C, palladium (10% on charcoal, 450 mg) was added with stirring to a solution of 6-chloro-2-trifluoromethoxy nicotinic acid (**37**, 1.5 g, 6.2 mmol) and ammonium formate (790 mg, 12.4 mmol, 2 eq) in methanol (10 mL). The reaction mixture was stirred for 16 h at 55 °C before being filtrated under suction and the filtrate evaporated. The residue was partitioned between ethyl acetate (2× 20 mL) and 2.0 M hydrochloric acid (30 mL). The combined organic layers were dried over sodium sulfate before being evaporated to afford pure 2-trifluoromethoxy nicotinic acid (**38**, 1.05 g, 5.0 mmol, 81 %) as a white powder; m.p. 82-85 °C.
¹H NMR (CD₃OD, 300 MHz): δ = 8.15 (dd, *J* = 4.6, 1.9 Hz, 1 H), 7.92 (dd, *J* = 7.6, 1.9 Hz, 1 H), 7.24 (dd, *J* = 7.6,4.6 Hz, 1 H). - ¹⁹F NMR ((CD₃)₂CO, 282 MHz): δ = -56.7 - ¹³C NMR (CD₃OD, 75 MHz): δ = 164.8, 154.2, 150.3, 142.2, 120.4 (q, *J* = 260 Hz), 121.9, 117.6. - C₇H₄F₃NO₃ (207): calcd. (%) C 40.59, H 1.95, N 6.76; found C 40.35, H 2.03, N 6.65.

### 5-amino-2-chloro-6-trifluoromethoxypyridine (39)

At 0 °C, diisopropylamine (1.7 g, 2.4 mL, 16.7 mmol, 1.1 eq) was added dropwise to a solution of butyllithium (1.56 M in hexane, 10.7 mL, 16.7 mmol, 1.1 eq) in THF (25 mL). At -78 °C, a solution of 2-chloro-6-trifluoromethoxypyridine (**2**, 3.0 g, 15.2 mmol, 1 eq) in THF (7 mL) was added dropwise followed after 2 h by benzenesulfonyl azide (3.3 g, 18.2 mmol, 1.2 eq). The reaction mixture was allowed to reach 25 °C before being treated with a saturated aqueous solution of ammonium chloride (30 mL) and extracted with diethylehter (3 × 20 mL). The combined organic layers were dried over sodium sulfate and evaporated to afford a crude red oil of 5-azido-2-chloro-6-trifluoromethoxy pyridine. It was then dissolved in anhydrous diethylether (100 mL) and added dropwise to a suspension of lithium aluminium hydride (690 mg, 18.2 mmol, 1.2 eq) in diehtylether (100 mL). The reaction mixture was heated under reflux for 5 h before being treated with water (100 mL) and extracted with diethylether (3 × 80 mL). The combined organic layers were dried over sodium sulfate before being evaporated. The crude product was purified by chromatography on silica gel using ethyl acetate/cyclohexane (3:7) as eluent which afforded pure 5-amino-2-chloro-6-trifluoromethoxy pyridine (**39**, 2.3 g, 10.8 mmol, 71%) as yellow crystals; m.p. 42-45 °C.
¹H NMR (CDCl₃, 300 MHz): δ = 6.98 (d, *J* = 8.2 Hz, 1 H), 6.95 (d, *J* = 8.2 Hz, 1 H), 3.83 (bs, 2 H). -¹⁹F NMR (CDCl₃, 282 MHz): δ = -56.5 -¹³C NMR (CDCl₃, 75 MHz): δ = 142.5, 135.3, 131.1, 126.3, 122.7, 120.1 (q, *J* = 262 Hz). - C₆H₄ClF₃N₂O (212): calcd. (%) C 33.90, H 1.90, N 13.18; found C 33.54, H 2.06, N 13.00.

### 3-amino-2-trifluoromethoxy pyridine (40)

At 25 °C, palladium (10% on charcoal, 730 mg) was added with stirring to a solution of 5-amino-2-chloro-6-trifluoromethoxypyridine (**39**, 1.6 g, 7.4 mmol) and ammonium formate (940 mg, 14.8 mmol, 2 eq) in methanol (12 mL). The reaction mixture was stirred for 16 h at 55 °C before being filtrated under suction and the solvent evaporated. The residue was partitioned between ethyl acetate (2× 20 mL) and water (30 mL). The combined organic layers were dried over sodium sulfate before being evaporated to afford pure 3-amino-2-trifluoromethoxypyridine (**40**, 1.05 g, 5.9 mmol, 80%) as a yellow oil.
¹H NMR (CDCl₃, 300 MHz): δ = 7.68 (dd, *J* = 4.6, 1.7 Hz, 1 H), 7.09 (dd, *J* = 7.8, 1.7 Hz 1 H), 7.02 (dd, *J* = 7.8, 4.6 Hz, 1 H), 3.90 (bs, 2 H). - ¹⁹F NMR (CDCl₃, 282 MHz): δ = -56.2 -¹³C NMR (CDCl₃, 75 MHz): δ = 144.6, 138.2, 132.2, 124.1, 122.4, 120.3 (q, *J* = 262 Hz). - C₆H₃F₃N₂O (178): calcd. (%) C 40.46, H 2.83, N 15.73; found C 41.28, H 2.90, N 16.02.

### 2-Chloro-5-iodo-6-trifluoromethoxy pyridine (41)

At 0 °C, diisopropylamine (2.2 g, 3.1 mL, 22.2 mmol, 1.1 eq) was added dropwise to a solution of butyllithium (1.56 M in hexane, 14.2 mL, 22.2 mmol, 1.1 eq) in THF (35 mL). At -78 °C, a solution of 2-chloro-6-trifluoromethoxypyridine (**2**, 4.0 g, 20.2 mmol, 1 eq) in THF (10 mL) was added dropwise followed after 2 h by a solution of iodide (5.7 g, 22.2 mmol, 1.1 eq) in THF (10 mL). The reaction mixture was allowed to reach 25 °C before being treated with a saturated aqueous solution of sodium sulfite (30 mL) and extracted with dichloromethane (3 × 20 mL). The combined organic layers were dried over sodium sulfate before being evaporated. The crude dark oil was distilled under vacuum (b.p. 103-106 °C / 16 mbar) to afford pure 2-chloro-5-iodo-6-trifluoromethoxypyridine (**41**, 5.1 g, 15.7 mmol, 78%) as colorless needles; m.p. 33-35 °C.
¹H NMR (CDCl₃, 300 MHz): δ = 8.11 (d, *J* = 8.1 Hz, 1 H), 7.03 (d, *J* = 8.1 Hz, 1 H). - ¹⁹F NMR (CDCl₃, 282 MHz): δ = -57.1 -¹³C NMR (CDCl₃, 75 MHz): δ = 154.9, 151.7, 148.8, 142.0, 123.3, 120.4 (q, *J* = 264 Hz).

### 2-Chloro-4-iodo-6-trifluoromethoxy pyridine (42)

At 0 °C, diisopropylamine (1.4 g, 1.9 mL, 13.6 mmol, 1.1 eq) was added dropwise to a solution of butyllithium (1.56 M in hexane, 9.0 mL, 13.6 mmol, 1.1 eq) in THF (20 mL). At -78 °C, a solution of 2-chloro-5-iodo-6-trifluoromethoxypyridine (**41**, 4.0 g, 12.4 mmol, 1 eq) in THF (10 mL) was added dropwise and the reaction mixture was stirred for 1 h at this temperature. It was then neutralized with a solution of hydrochloric acid (2N, 10 mL), treated with a saturated aqueous solution sodium hydrogencarbonate (30 mL) and extracted with diethylether (3 × 20 mL). The combined organic layers were dried over sodium sulfate before being evaporated. The crude dark oil was distilled under vacuum (b.p. 104-107 °C / 16 mbar) to afford pure 2-chloro-4-iodo-6-trifluoromethoxypyridine (**42**, 3.2 g, 10.9 mmol, 80%) as a colorless oil; ¹H NMR (CDCl₃, 300 MHz): δ = 7.66 (d, *J* = 1.0 Hz, 1 H), 7.34 (d, *J* = 1.0 Hz, 1 H). -¹⁹F NMR (CDCl₃, 282 MHz): δ = -57.1 - ¹³C NMR (CDCl₃, 75 MHz): δ = 155.1, 149.4, 130.7, 120.3, 119.1 (q, *J* = 262 Hz), 108.3.

### 2-Chloro-6-trifluoromethoxy isonicotinic acid (43)

At 0 °C, butyllithium (1.56 M in hexane, 4.0 mL, 6.2 mmol, 0.67 eq) was added dropwise to a solution of butylmagnesium chloride (2M in THF, 1.5 mL, 3.0 mmol, 0.33 eq) in THF (8 mL), followed after 10 min by a solution of 2-chloro-4-iodo-6-trifluoromethoxypyridine (**42**, 3.0 g, 9.3 mmol, 1 eq) in THF (5 mL). After 10 min the reaction mixture was poured onto an excess of freshly crushed dry ice before being treated with an aqueous solution of sodium hydroxide (5%, 15 mL). The resulting aqueous layer was collected, washed with diethylether (10 mL) and acidified to pH 4 by dropwise addition of hydrochloric acid (6 N, 5 mL). After extraction with ethyl acetate (3 × 10 mL), the combined organic layers were dried over sodium sulfate before being evaporated to afford pure 2-chloro-6-trifluoromethoxy isonicotinic acid (**43**, 1.6 g, 6.6 mmol, 71%) as a white powder; m.p. 65-68 °C.
¹H NMR (CDCl₃, 300 MHz): δ = 10.12 (br s, 1 H), 7.88 (d, *J* = 0.9 Hz, 1 H), 7.56 (d, *J* = 0.9 Hz, 1 H). -¹⁹F NMR (CDCl₃, 282 MHz): δ = -57.3 - ¹³C NMR (CDCl₃, 75 MHz): δ = 167.6, 156.3, 150.4, 143.0, 122.5, 120.2 (q, *J* = 260 Hz), 113.5. - C₇H₃ClF₃NO₃ (241): calcd. (%) C 34.81, H 1.25, N 5.80; found C 34.64, H 1.55, N 5.76.

### 2-Trifluoromethoxy isonicotinic acid (44)

At 25 °C, palladium (10% on charcoal, 330 mg) was added with stirring to a solution of 2-chloro-6-trifluoromethoxy isonicotinic acid (**43**, 1.1 g, 4.55 mmol) and ammonium formate (574 mg, 9.1 mmol, 2 eq) in methanol (8 mL). The reaction mixture was stirred for 16 h at 55 °C before being filtrated under suction and the filtrate evaporated. The residue was partitioned between ethyl acetate (2× 15 mL) and 2.0 M hydrochloric acid (20 mL). The combined organic layers were dried over sodium sulfate before being evaporated to afford pure 2-trifluoromethoxy isonicotinic acid (**44**, 760 mg, 3.7 mmol, 81 %) as a white powder; m.p. 149-152 °C.
¹H NMR (CD₃OD, 300 MHz): δ = 8.39 (d, *J* = 5.1 Hz, 1 H), 7.77 (dd, *J* = 5.1, 1.0 Hz, 1 H), 7.51 (d, *J* = 1.0 Hz, 1 H). - ¹⁹F NMR ((CD₃)₂CO, 282 MHz): δ = -57.6 - ¹³C NMR (CD₃OD, 75 MHz): δ = 164.9, 157.2, 148.4, 143.3, 121.8, 120.3 (q, *J* = 260 Hz), 112.5. - C₇H₄F₃NO₃ (207): calcd. (%) C 40.59, H 1.95, N 6.76; found C 40.28, H 2.21, N 6.67.

### 4-Amino-2-chloro-6-trifluoromethoxypyridine (45)

At -78 °C, butyllithium (1.56 M in hexane, 2.2 mL, 3.4 mmol, 1.1 eq) was added dropwise to a solution of 2-chloro-4-iodo-6-trifluoromethoxypyridine (**42**, 1.0 g, 3.1 mmol) in THF (8 mL) followed after 10 min by benzenesulfonyl azide (0.6 g, 3.4 mmol, 1.1 eq). The reaction mixture was was allowed to reach 25 °C before being treated with a saturated aqueous solution of ammonium chloride (10 mL) and extracted with diethylehter (3 × 8 mL). The combined organic layers were dried over sodium sulfate and evaporated to afford a crude red oil of 4-azido-2-chloro-6-trifluoromethoxypyridine. It was then dissolved in anhydrous diethylether (25 mL) and added dropwise to a suspension of lithium aluminium hydride (130 mg, 3.4 mmol, 1.1 eq) in diehtylether (25 mL). The reaction mixture was heated under reflux for 5 h before being treated with water (10 mL) and extracted with diethylether (3 × 10 mL). The combined organic layers were dried over sodium sulfate before being evaporated. The crude product was purified by chromatography on silica gel using ethyl acetate/cyclohexane (1:9) as eluent which afforded pure 4-amino-2-chloro-6-trifluoromethoxypyridine (**45**, 0.3 g, 1.4 mmol, 46%) as a yellow oil.
¹H NMR (CDCl₃, 300 MHz): δ = 6.41 (d, *J* = 1.6 Hz, 1 H), 6.07 (d, *J* = 1.6 Hz, 1 H), 4.30 (bs, 2 H). -¹⁹F NMR (CDCl₃, 282 MHz): δ = -56.8 -¹³C NMR (CDCl₃, 75 MHz): δ = 157.4, 156.7, 149.7, 119.9 (q, *J* = 260 Hz), 107.6, 96.0. - C₆H₄ClF₃N₂O (212): calcd. (%) C 33.90, H 1.90, N 13.18; found C 33.56, H 2.01, N 12.87.

### 4-Amino-6-trifluoromethoxypyridine (46)

At 25 °C, palladium (10% on charcoal, 220 mg) was added with stirring to a solution of 4-amino-2-chloro-6-trifluoromethoxypyridine (**45**, 600 mg, 2.8 mmol, 1 eq) and ammonium formate (350 mg, 5.6 mmol, 2 eq) in methanol (10 mL). The reaction mixture was stirred for 16 h at 55 °C before being filtrated under suction and the filtrate evaporated. The residue was partitioned between ethyl acetate (2× 10 mL) and an aqueous solution of sodium hydroxide (4%, 15 mL). The combined organic layers were dried over sodium sulfate before being evaporated. The crude oil was purified by chromatography on silica gel using ethyl acetate/cyclohexane (2 : 8) as eluent to afford pure 4-amino-6-trifluoromethoxypyridine (**46**, 390 mg, 2.2 mmol, 79%) as a colorless oil which crystallized on standing.
¹H NMR (CD₃OD, 300 MHz): δ = 7.74 (d, *J* = 5.9 Hz, 1H), 6.54 (dd, *J* =5.9, 1.9 Hz, 1 H), 6.30 (d, *J* = 1.9 Hz, 1 H), 4.35 (bs, 2 H). -¹⁹F NMR (CDCl₃, 282 MHz): δ = -56.6 - ¹³C NMR (CD₃OD, 75 MHz): δ = 160.3, 158.9, 148.2, 121.5 (q, *J* = 260 Hz), 109.7, 97.7.

### 2-Chloro-6-trifluoromethoxy-5-trimethylsilylpyridine (47)

At 0 °C, diisopropylamine (0.9 g, 1.2 mL, 8.9 mmol, 1.1 eq) was added dropwise to a solution of butyllithium (1.56 M in hexane, 5.7 mL, 8.9 mmol, 1.1 eq) in THF (15 mL). At -78 °C, a solution of 2-chloro-6-trifluoromethoxy pyridine (**2**, 1.6 g, 8.1 mmol, 1 eq) in THF (5 mL) was added dropwise and the reaction mixture was stirred for 2 h at this temperature. Chlorotrimethylsilane (1.0 g, 1.2 mL, 8.9 mmol, 1.1 eq) was then added and the mixture was allowed to reach 25 °C before being neutralized with water (20 mL) and extracted with diethylether (3 × 10 mL). The combined organic layers were dried over sodium sulfate before being evaporated. The crude product was distilled under vacuum to afford pure 2-chloro-6-trifluoromethoxy-5-trimethylsilylpyridine (**47**, 1.5 g, 5.5 mmol, 68%) as a colorless oil; b.p. 89-93 °C /14 mbar.
¹H NMR (CDCl₃, 300 MHz): δ = 7.78 (d, *J* = 7.6 Hz, 1 H), 7.22 (d, *J* = 7.6 Hz, 1 H), 0.34 (s, 9 H). - ¹³C NMR (CDCl₃, 75 MHz): δ = 159.5, 149.8, 147.7, 147.3, 121.5, 120.4 (q, *J* = 260 Hz), -1.7.

### 2-Chloro-3-iodo-6-trifluoromethoxypyridine (48)

At 0 °C, 2,2,6,6-tetramethylpiperidine (0.4 g, 0.5 mL, 2.8 mmol, 1.1 eq) was added dropwise to a solution of butyllithium (1.56 M in hexane, 1.8 mL, 2.8 mmol, 1.1 eq) in THF (6 mL). At -78 °C, a solution of 2-chloro-6-trifluoromethoxy-5-trimethylsilylpyridine (**47**, 0.7 g, 2.6 mmol) in THF (3 mL) was added dropwise followed after 3 h by a solution of iodine (0.7 g, 2.8 mmol, 1.1 eq) in THF (5 mL). The mixture was then treated with a saturated aqueous solution of sodium sulfite (5 mL) and extracted with dichloromethane (3 × 5 mL). The combined organic layers were dried over sodium sulfate before being evaporated. The crude oil was treated with tetrabutylammonium fluoride (1M in THF, 2.6 mL, 2.6 mmol, 1 eq) for 20 h at 25 °C. The mixture was neutralized by addition of hydrochloric acid (2N, 5 mL) and extracted with ethyl acetate (3 × 5 mL). The combined organic layers were dried over sodium sulfate before being evaporated. The crude oil was purified by distillation under vacuum (50-51 °C / 1 mbar) to afford pure 2-chloro-3-iodo-6-trifluoromethoxypyridine (**48**, 550 mg, 1.7 mmol, 65%) as a colorless oil.
¹H NMR (CDCl₃, 300 MHz): δ = 8.11 (s, *J* = 8.3 Hz, 1 H), 6.65 (s, *J* = 8.3 Hz, 1 H). - ¹⁹F NMR (CDCl₃, 282 MHz): δ = -57.4 - ¹³C NMR (CDCl₃, 75 MHz): δ = 154.3, 150.9, 129.7, 120.5, 118.2 (q, *J* = 260 Hz), 111.5.

### 2-Chloro-6-trifluoromethoxynicotinic acid (49)

At 0 °C, 2,2,6,6-tetramethylpiperidine (0.84 g, 1.0 mL, 5.7 mmol, 1.1 eq) was added dropwise to a solution of butyllithium (1.56 M in hexane, 3.65 mL, 5.7 mmol, 1.1 eq) in THF (10 mL). At -78 °C, a solution 2-chloro-6-trifluoromethoxy-5-trimethylsilylpyridine (**47**, 1.4 g, 5.2 mmol, 1 eq) in THF (5 mL) was added dropwise and the reaction mixture was stirred for 2 h at this temperature. The mixture was then poured onto an excess of freshly crushed dry ice before being treated with an aqueous solution of sodium hydroxide (5%, 10 mL). The resulting aqueous layer was collected, washed with diethylether (10 mL) and acidified to pH 4 by dropwise addition of hydrochloric acid (6 N, 4 mL). After extraction with ethyl acetate (3 × 10 mL), the combined organic layers were dried over sodium sulfate before being evaporated. The crude oil was treated with tetrabutylammonium fluoride (1M in THF, 5.7 mL, 5.7 mmol, 1.1 eq) for 20 h at 25 °C. The mixture was neutralized by addition of hydrochloric acid (2N, 10 mL) and extracted with ethyl acetate (3 × 10 mL). The combined organic layers were dried over sodium sulfate before being evaporated to afford pure 2-chloro-6-trifluoromethoxynicotinic acid (**49**, 880 mg, 3.65 mmol, 70%) as a white powder; m.p. 135-138 °C.
¹H NMR (CD₃OD, 300 MHz): δ = 8.44 (d, *J* = 8.3 Hz, 1 H), 7.21 (d, *J* = 8.3 Hz, 1 H). - ¹⁹F NMR ((CD₃)₂CO, 282 MHz): δ = -57.9 -¹³C NMR (CD₃OD, 75 MHz): δ = 164.9, 156.1, 147.9, 144.7, 125.5, 120.1 (q, *J* = 260 Hz), 110.8.

### 6-Trifluoromethoxynicotinic acid (50)

At 25 °C, palladium (10% on charcoal, 334 mg) was added with stirring to a solution of 2-chloro-6-trifluoromethoxy nicotinic acid (**49**, 835 mg, 3.45 mmol, 1 eq) and ammonium formate (435 mg, 6.9 mmol, 2 eq) in methanol (6 mL). The reaction mixture was stirred for 16 h at 55 °C before being filtrated under suction and the filtrate evaporated. The residue was partitioned between ethyl acetate (2× 10 mL) and 2.0 M hydrochloric acid (15 mL). The combined organic layers were dried over sodium sulfate before being evaporated to afford pure 6-trifluoromethoxynicotinic acid (**50**, 580 mg, 2.8 mmol, 81 %) as a white powder; m.p. 118-121 °C.
¹H NMR (CD₃OD, 300 MHz): δ = 8.72 (d, *J* = 2.3 Hz, 1 H), 8.30 (dd, *J* = 8.4, 2.3 Hz, 1 H), 7.04 (d, *J* = 8.4 Hz, 1 H). - ¹⁹F NMR (CDCl₃, 282 MHz): δ = -57.1 - ¹³C NMR (CD₃OD, 75 MHz): δ = 165.5, 159.2, 149.5, 141.8, 125.4, 120.2 (q, *J* = 260 Hz), 112.1.

### 3-Amino-2-chloro-6-trifluoromethoxypyridine (51)

At 0 °C, 2,2,6,6-tetramethylpiperidine (5.2 g, 6.2 mL, 36.7 mmol, 1.1 eq) was added dropwise to a solution of butyllithium (1.56 M in hexane, 23.5 mL, 36.7 mmol, 1.1 eq) in THF (60 mL). At -78 °C, a solution of 2-chloro-6-trifluoromethoxy-5-trimethylsilylpyridine (**47**, 9.0 g, 33.4 mmol) in THF (20 mL) was added dropwise followed after 3 h by a solution of benzenesulfonyl azide (6.7 g, 36.7 mmol, 1.1 eq). The reaction mixture was allowed to reach 25 °C before being treated with a saturated aqueous solution of ammonium chloride (50 mL) and extracted with diethylehter (3 × 40 mL). The combined organic layers were dried over sodium sulfate and evaporated to afford a crude red oil of 3-azido-2-chloro-6-trifluoromethoxypyridine. It was then dissolved in anhydrous diethylether (250 mL) and added dropwise to a suspension of lithium aluminium hydride (1.4 g, 37.0 mmol, 1.5 eq) in diehtylether (250 mL). The reaction mixture was heated under reflux for 5 h before being treated with water (100 mL) and extracted with diethylether (3 × 50 mL). The combined organic layers were dried over sodium sulfate before being evaporated. The crude oil was treated with tetrabutylammonium fluoride (1M in THF, 34 mL, 33.4 mmol, 1 eq) for 20 h at 25 °C. The mixture was neutralized by addition of sodium hydroxide (4%, 100 mL) and extracted with ethyl acetate (3 × 50 mL). The combined organic layers were dried over sodium sulfate before being evaporated. The crude product was purified by chromatography on silica gel using ethyl acetate/cyclohexane (5 : 95) as eluent which afforded pure 3-amino-2-chloro-6-trifluoromethoxypyridine (**51**, 4.5 g, 21.4 mmol, 64%) as yellow crystals; m.p. 58-61 °C. -¹H NMR (CDCl₃, 300 MHz): δ = 7.05 (d, *J* = 8.4 Hz, 1 H), 6.78 (d, *J* = 8.4 Hz, 1 H), 4.03 (bs, 2 H). -¹⁹F NMR (CDCl₃, 282 MHz): δ = -57.2 - ¹³C NMR (CDCl₃, 75 MHz): δ = 146.0, 138.8, 132.8, 125.9, 120.2 (q, *J* = 257 Hz), 113.6.

### 3-Amino-6-trifluoromethoxypyridine (52)

At 25 °C, palladium (10% on charcoal, 700 mg) was added with stirring to a solution of 3-amino-2-chloro-6-trifluoromethoxypyridine (**51**, 1.3 g, 6.1 mmol, 1 eq) and ammonium formate (770 mg, 12.1 mmol, 2 eq) in methanol (20 mL). The reaction mixture was stirred for 16 h at 55 °C before being filtrated under suction and the filtrate evaporated. The residue was partitioned between ethyl acetate (2× 30 mL) and an aqueous solution of sodium hydroxide (4%, 30 mL). The combined organic layers were dried over sodium sulfate before being evaporated to afford pure 3-amino-6-trifluoromethoxypyridine (**52**, 920 mg, 5.2 mmol, 85%) as colorless crystals; m.p. 31-33 °C.
¹H NMR (CDCl₃, 300 MHz): δ = 7.66 (d, *J* = 2.9 Hz, 1 H), 6.97 (dd, *J* = 8.6, 2.9 Hz, 1 H), 6.76 (d, *J* = 8.6, 1 H), 3.55 (bs, 2 H). - ¹⁹F NMR (CDCl₃, 282 MHz): δ = -57.7 - ¹³C NMR (CDCl₃, 75 MHz): δ = 148.8, 141.6, 133.9, 125.8, 120.3 (q, *J* = 257 Hz), 114.3. - C₆H₅F₃N₂O (178): calcd. (%) C 40.46, H 2.83, N 15.73; found C 40.00, H 2.92, N 15.37.

### 3-Bis(trimethylsilyl)amino-6-trifluoromethoxypyridine (53)

At -78 °C, butyllithium (1.56 M, 7.2 mL, 11.2 mmol, 2 eq) was added dropwise to a solution of 3-amino-6-trifluoromethoxypyridine (**52**, 1.0 g, 5.6 mmol) in THF (12 mL) followed after 1 min by chlorotrimethylsilane (1.4 mL, 11.2 mmol, 2 eq). The reaction mixture was allowed to reach 25 °C for 2 h before being filtrated onto a pad of celite. The solvent was removed and the crude oil was distilled under vacuum (101-103 °C / 1 mbar) to afford pure 3-*bis*(trimethylsilyl)amino-6-trifluoromethoxypyridine (**53**, 1.5 g, 4.6 mmol, 83%) as a colorless oil.
¹H NMR (CDCl₃, 300 MHz): δ = 7.80 (d, *J* = 2.7 Hz, 1 H), 7.21 (dd, *J* = 8.5, 2.7 Hz, 1 H), 6.81 (d, *J* = 8.5, 1 H), 0.00 (s, 18 H). -¹³C NMR (CDCl₃, 75 MHz): δ = 150.7, 146.3, 141.5, 139.1, 118.3 (q, *J* = 260 Hz), 110.8,0.02.

### 5-Amino-2-trifluoromethoxynicotinic acid (54)

At 0 °C, diisopropylamine (0.5 g, 0.7 mL, 4.6 mmol, 1.1 eq) was added dropwise to a solution of butyllithium (1.56 M in hexane, 3.0 mL, 4.6 mmol, 1 eq) in THF (10 mL). At -78 °C, a solution of 3-*bis*(trimethylsilyl)amino-6-trifluoromethoxypyridine (**53**, 1.4 g, 4.2 mmol) in THF (5 mL) was added dropwise and the reaction mixture was stirred for 3 h at this temperature. The mixture was then poured onto an excess of freshly crushed dry ice before being treated with an aqueous solution of sodium hydroxide (5%, 10 mL). The resulting aqueous layer was collected, washed with diethylether (5 mL) and acidified to pH 3 by dropwise addition of hydrochloric acid (6 N, 3 mL). After extraction with ethyl acetate (4 × 5 mL), the combined organic layers were dried over sodium sulfate before being evaporated to afford pure 6-amino-2-trifluoromethoxynicotinic acid (**54**, 450 mg, 2.9 mmol, 69%) as a white powder; m.p. 161-163 °C
¹H NMR (CD₃OD, 300 MHz): δ = 7.70 (d, *J* = 3.0 Hz, 1H), 7.51 (d, *J* = 3.0, 1 H), 6.76 (d, *J* = 8.6, 1 H), 3.55 (bs, 2 H). -¹⁹F NMR (CDCl₃, 282 MHz): δ = -56.4 - ¹³C NMR (CD₃OD, 75 MHz): δ = 166.9, 146.0, 145.8, 136.8, 127.2, 121.5 (q, *J* = 260 Hz), 120.4. - C₇H₅F₃N₂O₃ (222): calcd. (%) C 37.89, H 2.27, N 12.61; found C 37.59, H 2.76, N 12.66.

### 2-Bromo-6-trifluoromethoxypyridine (55)

A solution of 2-chloro-6-trifluoromethoxypyridine (**2**, 7.0 g, 35.4 mmol) in hydrobromic acid (33% in acetic acid, 100 mL) was heated for 3 days at 100 °C. The reaction mixture was cooled down to 0 °C before being slowly neutralized by addition of saturated aqueous solution of sodium hydrogencarbonate (500 mL). After extraction with ethyl acetate (4 × 100 mL), the combined organic layers were dried over sodium sulfate before being evaporated. The crude oil was distilled under vacuum (67-69 °C / 15 mbar) to afford pure 2-bromo-6-trifluoromethoxypyridine (**2**, 4.2 g, 17.3 mmol, 48%) as a colorless oil.
¹H NMR (CDCl₃, 300 MHz): δ = 7.58 (t, *J* = 7.9 Hz, 1 H), 7.37 (d, *J* = 7.7, 1 H), 6.92 (d, *J* = 8.0, 1 H). -¹⁹F NMR (CDCl₃, 282 MHz): δ = -56.5 -¹³C NMR (CDCl₃, 75 MHz): δ 154.5, 141.0, 138.1, 125.1, 119.0 (q, *J* = 260 Hz), 110.1.

### 6-Trifluoromethoxypicolinic acid (56)

At -78 °C, butyllithium (1.56 M in hexane, 0.8 mL, 1.2 mmol, 1 eq) was added dropwise to a solution 2-bromo-6-trifluoromethoxypyridine (**55**, 0.3 g, 1.2 mmol) in dried toluene (4 mL). After 40 min at -78 °C, the mixture was poured onto an excess of freshly crushed dry ice before being treated with an aqueous solution of sodium hydroxide (5%, 4 mL). The resulting aqueous layer was collected, washed with diethylether (4 mL) and acidified to pH 3 by dropwise addition of hydrochloric acid (6 N, 1 mL) before being extracted with ethyl acetate (3 × 4 mL). The combined organic layers were dried over sodium sulfate and evaporated to afford pure 6-trifluoromethoxypicolinic acid (**56**, 150 mg, 0.7 mmol, 60%) as a white powder; m.p. 126-128 °C.
¹H NMR (CDCl₃, 300 MHz): δ = 8.10 (d, *J* = 7.4 Hz, 1 H), 8.00 (t, *J* = 7.8, 1 H), 7.25 (d, *J* = 8.1, 1 H). -¹⁹F NMR ((CD₃)₂CO, 282 MHz): δ = -57.6 - ¹³C NMR (CDCl₃, 75 MHz): δ = 162.9, 155.4, 144.6, 142.6, 122.1, 120.4 (q, *J* = 260 Hz), 117.5.

### 2-Amino-6-trifluoromethoxypyridine (57)

2-Bromo-6-trifluoromethoxypyridine (**55**, 500 mg, 2.1 mmol), benzophenone imine (455 mg, 2.5 mmol, 1.2 eq), KOtBu (360 mg, 3.1 mmol, 1.5 eq), DPEphos (25 mg, 0.04 mmol, 0.02 eq), Pd₂dba₃ (60 mg, 0.08 mmol, 0.04 eq) were introduced in dry toluene (6 mL) and the reaction mixture was heated at 70 °C and vigorously stirred for 2 h. GC monitoring indicated 100% conversion. The mixture was allowed to reach 25 °C before being filtrated on celite and washed with ethyl acetate. The filtrate was poured onto a 10% aqueous solution of citric acid (10 mL) and the reaction mixture was then vigorously stirred for 16 h at room temperature. GC of the organic phase indicated disappearance of starting reagent and formation of diphenylketone. The aqueous phase was adjusted to pH 9-10 with 5% sodium hydroxide (7 mL) and extracted with ethyl acetate (5 × 5 mL). The combined organic layers were dried over sodium sulfate before being evaporated. The crude oil was purified by chromatography on silica gel using ethyl acetate/cyclohexane (5 : 95) as eluent which afforded pure 2-amino-6-trifluoromethoxypyridine (**57**, 150 mg, 0.8 mmol, 40%) as a yellow oil.
¹H NMR (CDCl₃, 300 MHz): δ = 7.40 (t, *J* = 7.9 Hz, 1 H), 6.29 (d, *J* = 7.8, 1 H), 6.27 (d, *J* = 7.3, 1 H), 4.19 (br s, 2 H). - ¹⁹F NMR (CDCl₃, 282 MHz): δ = -56.7 - ¹³C NMR (CDCl₃, 75 MHz): δ = 157.6, 155.6, 141.2, 120.2 (q, *J* = 260 Hz), 105.8, 101.2. - C₆H₅F₃N₂O (178): calcd. (%) C 40.46, H 2.73, N 15.73; found C 40.74, H 3.12, N 16.10.

### Functionalized 4-OCF₃-pyridine

### 2-Chloro-4-trifluoromethoxynicotinic acid (58)

At 0 °C, diisopropylamine (1.0 g, 1.4 mL, 9.7 mmol, 1.1 eq) was added dropwise to a solution of butyllithium (1.42 M in hexane, 6.8 mL, 9.7 mmol, 1.1 eq) in THF (25 mL). At -78 °C, a solution of 2-chloro-4-trifluoromethoxypyridine (**10**, 1.7 g, 8.8 mmol, 1 eq) in THF (5 mL) was added dropwise and the reaction mixture was stirred for 2 h at this temperature. The mixture was then poured onto an excess of freshly crushed dry ice before being treated with an aqueous solution of sodium hydroxide (5%, 25 mL). The resulting aqueous layer was collected, washed with diethylether (15 mL) and acidified to pH 4 by dropwise addition of hydrochloric acid (6 N, 8 mL). After extraction with ethyl acetate (3 × 20 mL), the combined organic layers were dried over sodium sulfate and evaporated. Crystallization from a 3:1 mixture of hexanes and ethyl acetate afforded pure 2-chloro-4-trifluoromethoxy nicotinic acid (**58**, 1.7 g, 7.0 mmol, 80%) as colorless crystals; m.p. 111-114 °C.
¹H NMR (CDCl₃, 300 MHz): δ = 9.29 (br s, 1 H), 8.51 (d, *J* = 5.9 Hz, 1 H), 7.21 (dq, *J* = 5.9, 2.0 Hz, 1 H). - ¹⁹F NMR (CDCl₃, 282 MHz): δ = -57.7 - ¹³C NMR (CDCl₃, 75 MHz): δ = 165.1, 154.4, 151.5, 149.6, 122.1, 119.9 (q, *J* = 261 Hz), 112.2. - C₇H₃ClF₃NO₃ (241): calcd. (%) C 34.81, H 1.25, N 5.80; found C 34.68, H 1.56, N 5.90.

## Claims

1. A process for the preparation of functionalized trihalomethoxypyridines of formula (I) 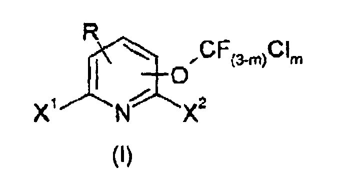 wherein
X¹ and X² are each individually selected from hydrogen, fluorine, chlorine or bromine, wherein at least one of X¹ and X² is different from hydrogen;
R is selected from hydrogen, halogen, C₁₋₁₂-alkyl-, C₃₋₈-cycloalkyl-, C₂₋₁₂-alkenyl-, C₂₋₁₂-alkynyl-, C₆₋₁₄aryl-, C₇₋₁₉-arylalkyl- oder C₇₋₁₉-alkylaryl-groups, wherein each of the alkyl- cycloalkyl-, alkenyl-, alkynyl-, aryl-, aralkyl- and alkyryl-groups can be substituted by further residues selected from the group consisting of -R', -X, -OR', -SR', -NR'₂, -SiR'₃, -COOR', -CN and -CONR₂', wherein R' is hydrogen or a C₁₋₁₂-alkyl-residue
m is 0,
comprising the following steps (A) to (C):
(A) reacting hydroxypyridines according to formula (II) with thiophosgene in the presence of a base 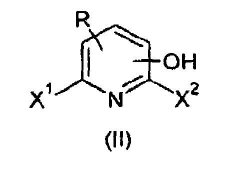
(B) reacting chlorothionoformiates of formula (III) with elemental chlorine; 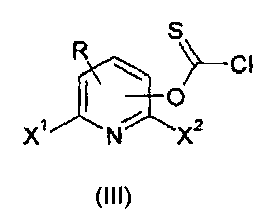
(C) treating the trichloromethoxy pyridines of the formula (IV) with a fluoride source to obtain trihalomethoxy pyridines of the formula (I)

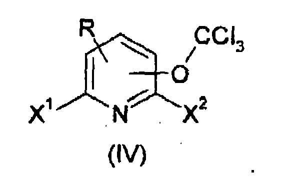

2. Process according to claim 1, wherein the halo-substituent is chlorine.

3. Process according to any one of claims 1 to 2, wherein the fluoride source in the chlorine/fluorine exchange step (C) is selected from SbF₃ , HF, HF*pyridine adduct, HF*amine adduct, KF, MoF₆, CoF₃, SbF₅.

4. Process according to any one of claims 1 to 3, wherein step (C) is carried out in presence of a catalyst.

5. Process according to claim 4, wherein the catalyst is SbCl₅ or BF₃.

6. Process according to any one of claims 1 to 5, wherein the chlorine/fluorine exchange step (C) is performed without any solvent.

7. Process according to any one of claims 1 to 5, wherein the chlorine/fluorine exchange step (C) is performed in sulfolane.

8. Process according to any one of claims 1 to 7 wherein the ratio of fluoride source and catalyst is in the range of 2:1 to 20:1.

## Patentansprüche

1. Verfahren zur Herstellung von funktionalisierten Trihalogenmethoxypyridinen der Formel (I) 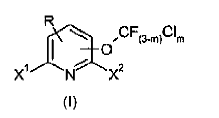 worin
X¹ und X² jeweils unabhängig voneinander unter Wasserstoff, Fluor, Chlor oder Brom ausgewählt sind, wobei mindestens eine der Gruppen X¹ und X² von Wasserstoff verschieden ist;
R unter Wasserstoff, Halogen, C₁₋₁₂-Alkyl-, C₃₋₈-Cycloalkyl, C₂₋₁₂-Alkenyl-, C₂₋₁₂-Alkinyl-, C₆₋₁₄-Aryl-, C₇₋₁₉-Arylalkyl-oder C₇₋₁₉-Alkylarylgruppen ausgewählt ist, wobei jede der Alkyl-, Cycloalkyl-, Alkenyl-, Alkinyl-, Aryl-, Aralkyl- und Alkarylgruppen durch weitere Reste, die aus der Gruppe bestehend aus -R', -X, -OR', -SR', -NR'₂, -SiR'₃, -COOR', -CN und -CONR'₂, worin R' für Wasserstoff oder einen C₁₋₁₂-Alkylrest steht, ausgewählt sind, substituiert sein kann;
m für 0 steht,
mit den folgenden Schritten (A) bis (C):
(A) Umsetzung von Hydroxypyridinen der Formel (II) mit Thiophosgen in Gegenwart einer Base 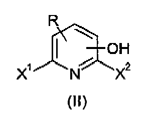
(B) Umsetzung von Chlorthionoformiaten der Formel (III) mit elementarem Chlor 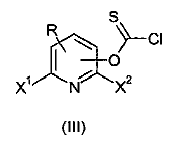
(C) Behandeln der Trichlormethoxypyridine der Formel (IV) mit einer Fluoridquelle zum Erhalt von Trihalogenmethoxypyridinen der Formel (I)

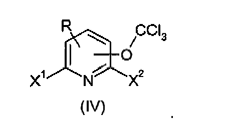

2. Verfahren nach Anspruch 1, bei dem es sich bei dem Halogensubstituenten um Chlor handelt.

3. Verfahren nach einem der Ansprüche 1 bis 2, bei dem man die Fluoridquelle bei dem Chlor/Fluor-Austauschschritt (C) unter SbF₃, HF, HF*Pyridin-Addukt, HF*Amin-Addukt, KF, MoF₆, CoF₃ und SbF₅ auswählt.

4. Verfahren nach einem der Ansprüche 1 bis 3, bei dem man Schritt (C) in Gegenwart eines Katalysators durchführt.

5. Verfahren nach Anspruch 4, bei dem es sich bei dem Katalysator um SbCl₅ oder BF₃ handelt.

6. Verfahren nach einem der Ansprüche 1 bis 5, bei dem man den Chlor/Fluor-Austauschschritt (C) ohne Lösungsmittel durchführt.

7. Verfahren nach einem der Ansprüche 1 bis 5, bei dem man den Chlor/Fluor-Austauschschritt (C) in Sulfolan durchführt.

8. Verfahren nach einem der Ansprüche 1 bis 7, bei dem das Verhältnis von Fluoridquelle zu Katalysator im Bereich von 2:1 bis 20:1 liegt.

## Revendications

1. Procédé de préparation de trihalogénométhoxypyridines fonctionnalisées de formule (I) 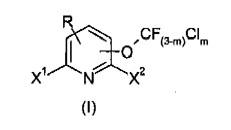 dans laquelle
X¹ et X² sont choisis chacun individuellement parmi hydrogène, fluor, chlore ou brome, où au moins l'un parmi X¹ et X² est différent d'hydrogène ;
R est choisi parmi hydrogène, halogène, des groupements C₁₋₁₂alkyl-, C₃₋₈cycloalkyl-, C₂₋₁₂alcényl-, C₂₋₁₂alcynyl-, C₆₋₁₄aryl-, C₇₋₁₉arylalkyl-, ou C₇₋₁₉alkylaryl-, où chacun parmi les groupements alkyl-, cycloalkyl-, alcényl-, alcynyl-, aryl-, aralkyl- et alkylaryl- peut être substitué par d'autres restes choisis dans le groupe constitué par -R', -X, -OR', -SR', -NR'₂, -siR'₃, -COOR', -CN et -CONR₂', où R' est hydrogène ou un reste C₁₋₁₂alkyl- ;
m vaut 0 ;
comprenant les étapes (A) à (C) suivantes :
(A) la réaction d'hydroxypyridines répondant à la formule (II) avec du thiophosgène en présence d'une base 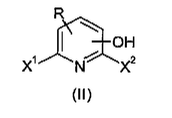
(B) la réaction de chlorothionoformiates de formule (III) avec du chlore élémentaire : 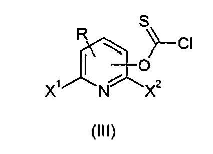
(C) le traitement des trichlorométhoxypyridines de formule (IV) par une source de fluor afin d'obtenir les trihalogénométhoxypyridines de formule (I)

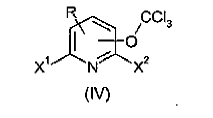

2. Procédé selon la revendication 1, dans lequel le substituant halogéno est le chlore.

3. Procédé selon l'une quelconque des revendications 1 à 2, dans lequel la source de fluor dans l'étape d'échange chlore/fluor (C) est choisie parmi SbF₃, HF, un produit d'addition HF*pyridine, un produit d'addition HF*amine, KF, MoF₆, CoF₃, SbF₅.

4. Procédé selon l'une quelconque des revendications 1 à 3, dans lequel l'étape (C) est réalisée en présence d'un catalyseur.

5. Procédé selon la revendication 4, dans lequel le catalyseur est SbCl₅ ou BF₃.

6. Procédé selon l'une quelconque des revendications 1 à 5, dans lequel l'étape d'échange chlore/fluor (C) est effectuée sans aucun solvant.

7. Procédé selon l'une quelconque des revendications 1 à 5, dans lequel l'étape d'échange chlore/fluor (C) est effectuée dans du sulfolane.

8. Procédé selon l'une quelconque des revendications 1 7, dans lequel le rapport de la source de fluor au catalyseur se trouve dans la plage allant de 2:1 à 20:1.
